(19) Europäisches Patentamt
European Patent Office
Office européen des brevets

(11) EP 0 668 104 B1

## (12) EUROPÄISCHE PATENTSCHRIFT

(45) Veröffentlichungstag und Bekanntmachung des Hinweises auf die Patenterteilung:
**19.05.1999 Patentblatt 1999/20**

(51) Int. Cl.$^6$: **B01J 23/88**, B01J 27/199

(21) Anmeldenummer: **95101967.8**

(22) Anmeldetag: **14.02.1995**

(54) **Multimetalloxidmassen.**

Multimetal oxide masses

Masses d'oxydes multimétalliques

(84) Benannte Vertragsstaaten:
**BE DE ES FR GB NL**

(30) Priorität: **22.02.1994 DE 4405514
17.11.1994 DE 4440891**

(43) Veröffentlichungstag der Anmeldung:
**23.08.1995 Patentblatt 1995/34**

(73) Patentinhaber:
**BASF Aktiengesellschaft
67063 Ludwigshafen (DE)**

(72) Erfinder:
• **Tenten, Andreas, Dr.
D-67434 Neustadt (DE)**

• **Martin, Friedrich-Georg, Dr.
D-69115 Heidelberg (DE)**
• **Hibst, Hartmut, Dr.
D-69198 Schriesheim (DE)**
• **Marosi, Laszlo, Dr.
D-67063 Ludwigshafen (DE)**
• **Kohl, Veronika, Dr.
D-64295 Darmstadt (DE)**

(56) Entgegenhaltungen:
EP-A- 0 000 835    EP-A- 0 358 411
EP-A- 0 467 144    EP-A- 0 575 897
FR-A- 2 534 904    GB-A-  694 354
US-A- 4 035 262

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die Erfindung betrifft Multimetalloxidmassen der allgemeinen Formel I

$$[A]_p \, [B]_q \qquad\qquad (I),$$

in der die Variablen folgende Bedeutung haben:

A $\quad$ $Mo_{12} \, V_a \, X_b^1 \, X_c^2 \, X_d^3 \, X_e^4 \, X_f^5 \, X_g^6 \, O_x$ $\qquad$ (Co-Phase),

B $\quad$ $X_{12}^7 \, Cu_h \, H_i \, O_y$ $\qquad$ (Schlüsselphase),

$X^1$ $\quad$ W, Nb, Ta, Cr und/oder Ce, vorzugsweise W, Nb und/oder Cr,

$X^2$ $\quad$ Cu, Ni, Co, Fe, Mn und/oder Zn, vorzugsweise Cu, Ni, Co und/oder Fe,

$X^3$ $\quad$ Sb und/oder Bi, vorzugsweise Sb,

$X^4$ $\quad$ Li, Na, K, Rb, Cs und/oder H, vorzugsweise Na und/oder K,

$X^5$ $\quad$ Mg, Ca, Sr und/oder Ba, vorzugsweise Ca, Sr und/oder Ba,

$X^6$ $\quad$ Si, Al, Ti und/oder Zr, vorzugsweise Si, Al und/oder Ti,

$X^7$ $\quad$ Mo, W, V, Nb und/oder Ta, vorzugsweise Mo,

a $\quad$ 1 bis 8, vorzugsweise 3 bis 6,

b $\quad$ 0,2 bis 5, vorzugsweise 0,5 bis 2,5,

c $\quad$ 0 bis 23, vorzugsweise 0 bis 4,

d $\quad$ 0 bis 50, vorzugsweise 0 bis 3,

e $\quad$ 0 bis 2, vorzugsweise 0 bis 0,3,

f $\quad$ 0 bis 5, vorzugsweise 0 bis 2,

g $\quad$ 0 bis 50, vorzugsweise 0 bis 20,

h $\quad$ 4 bis 30, vorzugsweise 6 bis 24, besonders bevorzugt 9 bis 18,

i $\quad$ 0 bis 20, vorzugsweise 0 bis 10,

x, y $\quad$ Zahlen, die durch die Wertigkeit und Häufigkeit der von Sauerstoff verschiedenen Elemente in I bestimmt werden und

p, q $\quad$ von Null verschiedene Zahlen, deren Verhältnis p/q 160:1 bis 1:1, vorzugsweise 20:1 bis 1:1 und besonders bevorzugt 15:1 bis 4:1 beträgt,

die den Anteil $[A]_p$ in Form dreidimensional ausgedehnter, von ihrer lokalen Umgebung aufgrund ihrer von ihrer lokalen Umgebung verschiedenen chemischen Zusammensetzung abgegrenzter, Bereiche A der chemischen Zusammensetzung

A $\quad$ $Mo_{12} \, V_a \, X_b^1 \, X_c^2 \, X_d^3 \, X_e^4 \, X_f^5 \, X_g^6 \, O_x$

und den Anteil $[B]_q$ in Form dreidimensional ausgedehnter, von ihrer lokalen Umgebung aufgrund ihrer von ihrer lokalen Umgebung verschiedenen chemischen Zusammensetzung abgegrenzter, Bereiche B der chemischen Zusammensetzung

B $\quad$ $X_{12}^7 \, Cu_h \, H_i \, O_y$

enthalten, wobei die Bereiche A, B relativ zueinander wie in einem Gemisch aus feinteiligem A und feinteiligem B verteilt sind.

Außerdem betrifft vorliegende Erfindung Verfahren zur Herstellung dieser Massen sowie deren Verwendung.

Die DE-A 4 335 973 und die US-A 4 035 262 betreffen Multimetalloxidmassen, deren Element-Bruttozusammensetzung derjenigen der erfindungsgemäßen Multimetalloxidmassen entspricht. Die Herstellung dieser Multimetalloxidmassen erfolgt dadurch, daß man geeignete Quellen der Bestandteile der gewünschten Multimetalloxidmassen in den erforderlichen Mengen zu einem innigen Trockengemisch verarbeitet und dieses anschließend bei erhöhter Temperatur mehrere Stunden calciniert. Die resultierenden Multimetalloxidmassen werden als Katalysatoren zur gasphasenkatalytisch oxidativen Herstellung von Acrylsäure aus Acrolein empfohlen. Nachteilig an den Multimetalloxidmassen dieses Standes der Technik ist jedoch, daß bei ihrer Verwendung die Selektivität der Acrylsäurebildung bei vorgegebenem Acroleinumsatz nicht voll zu befriedigen vermag. Ferner weisen diese Multimetalloxidmassen ein ausgeprägtes Formierungsverhalten auf. D.h. bei Verwendung von frisch hergestellten Multimetalloxidmassen erreicht die Selektivität der Acrylsäurebildung (bei vorgegebenem Acroleinumsatz) erst nach längerer Betriebsdauer ihren dann im wesentlichen stationären Endwert. Auch vermag die Reproduzierbarkeit ihrer Herstellung bezüglich des stationären Endwertes der Selektivität der Acrylsäurebildung nicht zu befriedigen.

Die EP-A 835, die DE-C 3 338 380, die DE-A 4 220 859 und die ältere Anmeldung DE-A 4 307 381 betreffen ebenfalls als Katalysatoren für die gasphasenkatalytisch oxidative Herstellung $\alpha,\beta$-monoethylenisch ungesättigter Carbonsäuren geeignete Multimetalloxidmassen, die in vorteilhafter Weise gleichfalls einen Schlüsselphase/Co-Phase-Aufbau aufweisen. Zwar umfassen die allgemeinen Formeln dieses Standes der Technik innerhalb einer breiten Mannigfaltigkeit möglicher Multimetalloxidmassen formal auch solche, deren Schlüsselphase neben Elementen wie Molybdän oder Wolfram gleichzeitig das Element Kupfer enthalten können, die Gesamtheit aller Ausführungsbeispiele umfaßt jedoch kein einziges solches Ausführungsbeispiel, vielmehr sind selbige auf solche beschränkt, deren Schlüsselphasen anstelle des Elements Kupfer das Element Wismut enthalten. Diese Ausführungsform empfiehlt der Stand der Technik nachdrücklich als die besonders bevorzugte. Nachteilig an dieser bevorzugten Ausführungsform des Standes der Technik ist jedoch, daß auch sie als Katalysator für die katalytische Gasphasenoxidation von Acrolein zu Acrylsäure hinsichtlich der Selektivität der Acrylsäurebildung bei vorgegebenem Acroleinumsatz nicht voll zu befriedigen vermag.

Aufgabe der vorliegenden Erfindung war daher, Multimetalloxidmassen zur Verfügung zu stellen, die die Nachteile der Multimetalloxidmassen des Standes der Technik nicht aufweisen. Demgemäß wurden die eingangs definierten Massen I gefunden.

Ganz besonders bevorzugte Massen I sind solche, deren Bereiche A eine Zusammensetzung gemäß der nachfolgenden allgemeinen Formel II aufweisen

$$Mo_{12}V_{a'}X_{b'}^1{}'X_{c'}^2{}'X_{f'}^5{}'X_{g'}^6{}'O_{x'}' \qquad (II),$$

mit

$X^1$     W und/oder Nb,
$X^2$     Cu und/oder Ni,
$X^5$     Ca und/oder Sr,
$X^6$     Si und/oder Al,
a'     2 bis 6,
b'     1 bis 2,
c'     1 bis 3,
f'     0 bis 0,75,
g'     0 bis 10 und
x'     eine Zahl, die durch die Wertigkeit und Häufigkeit der von Sauerstoff verschiedenen Elemente in II bestimmt wird.

Ferner ist es von Vorteil, wenn der Anteil $[B]_q$ der erfindungsgemäßen Multimetalloxidmassen in den letzteren in Form dreidimensional ausgedehnter Bereiche der chemischen Zusammensetzung B enthalten ist, deren Größtdurchmesser $d_B$ (längste durch den Schwerpunkt des Bereichs gehende Verbindungsstrecke zweier auf der Oberfläche (Grenzfläche) des Bereichs befindlicher Punkte) > 0 bis 300 µm, vorzugsweise 0,05 bis 200 µm, besonders bevorzugt 0,1 bis 50 µm und ganz besonders bevorzugt 0,1 bis 30 µm betragen. Selbstverständlich können die Größtdurchmesser aber auch 50 bis 150 µm oder 75 bis 125 µm betragen (die experimentelle Ermittlung der Größtdurchmesser gestattet z.B. die Methode der energiedispersiven Röntgenanalyse (EDXS), z.B. mittels einer Elektronenstrahl-Mikrosonde JEOL JCXA/733).

Die Anteile $[A]_p$, $[B]_q$ können in den erfindungsgemäßen Multimetalloxidmassen jeweils amorph und/oder kristallin vorliegen. Der Anteil $[B]_q$ liegt vorzugsweise kristallin vor. Dabei sind solche Multimetalloxidmassen bevorzugt, deren Bereiche B im wesentlichen aus Kristalliten bestehen, die das Röntgenbeugungsmuster (den Strukturtyp) wenigstens eines der nachfolgenden Kupfermolyb-date aufweisen (der Ausdruck in Klammern gibt die Quelle für den zugehörigen Röntgenbeugungsfingerabdruck wieder):

$Cu_3(MoO_4)_2(OH)_2$ (Lindgrenit, Karteikarte 36-405 der JCPDS-ICDD Kartei (1991)),
$Cu_4Mo_6O_{20}$ (A. Moini et al., Inorg. Chem. 25 (21) (1986) S. 3782 bis 3785),
$Cu_4Mo_5O_{17}$ (Karteikarte 39-181 der JCPDS-ICDD Kartei (1991)),
$Cu_6Mo_5O_{18}$ (Karteikarte 40-865 der JCPDS-ICDD Kartei (1991)),
$Cu_6Mo_4O_{15}$ (Karteikarte 35-17 der JCPDS-ICDD Kartei (1991)),
$Cu Mo O_4$ (Karteikarte 22-242 der JCPDS-ICDD Kartei (1991)),
$CuMoO_4$ (Russian Journal of Inorganic Chemistry 36 (7), 1991, S. 927-928, Table 1, $CuMoO_4$-III),
$Cu_{4-x}Mo_3O_{12}$ mit x=0 bis 0,25 (Karteikarte 24-56 und 26-547 der JCPDS-ICDD Kartei (1991)),
$Cu_3Mo_2O_9$ (Karteikarte 24-55 und 34-637 der JCPDS-ICDD Kartei (1991)),
$Cu_2Mo O_5$ (Karteikarte 22-607 der JCPDS-ICDD Kartei (1991)).

Vorzugsweise besteht der Anteil $[B]_q$ der erfindungsgemäßen Multimetalloxidmassen aus wenigstens einem dieser Kupfermolybdate selbst.

Günstige erfindungsgemäße Multimetalloxidmassen sind auch solche, deren Bereiche B Kristallite von Oxometallaten der allgemeinen Formel III

$$Cu\ Mo_A\ W_B\ V_C\ Nb_D\ Ta_E\ O_Y \cdot (H_2O)_F \qquad (III),$$

mit

$1/(A+B+C+D+E)$   0,7 bis 1,3, vorzugsweise 0,85 bis 1,15, besonders bevorzugt 0,95 bis 1,05 und ganz besonders bevorzugt 1,

F          0 bis 1,

$B+C+D+E$       0 bis 1, oder 0 bis 0,5, oder 0 bis 0,1 und

Y          eine Zahl, die durch die Wertigkeit und Häufigkeit der von Sauerstoff verschiedenen Elemente in III bestimmt wird,

des Strukturtyps enthalten, der durch die Verbindung $CuMoO_4$-III in Russian Journal of Inorganic Chemistry 36 (7), 1991 auf S. 927 in Table 1 definiert wird. Hier wird dieser Strukturtyp als Wolframit bezeichnet. Die Überprüfung auf Vorhandensein dieses Strukturtyps erfolgt anhand des Röntgenbeugungsmusters.

Die GB-A-694 354 offenbart mit der Verbindung $CuWO_4$ einen weiteren Vertreter dieses Strukturtyps.

Nachfolgend sollen Kristallite aus Oxometallaten III des Wolframit-Strukturtyps als Kristallite B* gekennzeichnet werden.

Gemäß den ebenda gemachten Ausführungen sind demnach solche Kristallite B* geeignet, die die Stöchiometrie

$$Cu\ Mo_A\ W_B\ V_c\ O_y \qquad (IV),$$

mit

$1/A+B+C$    0,7 bis 1,3, vorzugsweise 0,85 bis 1,15, besonders bevorzugt 0,95 bis 1,05 und ganz besonders bevorzugt 1, und

A, B, C     alle > 0, mit der Maßgabe, daß $B+C \leq 1$,

aufweisen.

Ferner eignen sich Kristallite B*, die die Stöchiometrie

$$Cu\ Mo_A\ W_B\ O_y \qquad (V),$$

mit

$1/A+B$      0,7 bis 1,3, vorzugsweise 0,85 bis 1,15, besonders bevorzugt 0,95 bis 1,05 und ganz besonders bevorzugt 1 und

A,B        alle > 0, mit der Maßgabe, daß $B \leq 1$,

aufweisen.

Auch eignen sich Kristallite B*, die die Stöchiometrie

$$Cu\ Mo_A\ V_c\ O_y \qquad (VI),$$

mit

$1/A+C$      0,7 bis 1,3, vorzugsweise 0,85 bis 1,15, besonders bevorzugt 0,95 bis 1,05 und ganz besonders bevorzugt 1 und

A,C        alle > 0, mit der Maßgabe, daß $C \leq 1$,

aufweisen.

Y ist in allen vorgenannten Fällen eine Zahl, die durch die Wertigkeit und Häufigkeit der von Sauerstoff verschiedenen Elemente bestimmt wird.

Weiterhin eignen sich Kristallite B*, die die Stöchiometrie CuMoO$_4$ aufweisen.

Je größer der Anteil der Kristallite B* am gesamten Anteil [B]$_q$ der erfindungsgemäßen Multimetalloxidmassen ist, desto vorteilhaftere erfindungsgemäße Multimetalloxidmassen liegen vor. Mit Vorteil beläuft sich der Anteil der Kristallite B*, bezogen auf die Gesamtmasse des Anteils [B]$_q$, auf wenigstens 5, besser wenigstens 10, noch besser wenigstens 25 Gew.-%. Vorzugsweise beträgt der vorgenannte Anteil wenigstens 50 Gew.-%, noch besser wenigstens 75 Gew.-% und ganz besonders bevorzugt wenigstens 90 Gew.-%.

Geeignet ist selbstverständlich auch ein Gewichtsanteil von 95 bis 100 Gew.-%.

Die erfindungsgemäßen Massen I sind in einfacher Weise z.B. dadurch erhältlich, daß man eine Multimetalloxidmasse

$$X_{12}^7 \; Cu_h \, H_i \, O_y \qquad\qquad (B)$$

in feinteiliger Form getrennt vorbildet (Ausgangsmasse 1) und anschließend die Ausgangsmasse 1 mit geeigneten Quellen der elementaren Konstituenten der Multimetalloxidmasse A

$$Mo_{12} \, V_a \, X_b^1 \, X_c^2 \, X_d^3 \, X_e^4 \, X_f^5 \, X_g^6 \, O_x \qquad\qquad (A)$$

im gewünschten Mengenverhältnis in innigen Kontakt bringt und ein daraus resultierendes Trockengemisch bei einer Temperatur von 250 bis 450°C calciniert, wobei die Calcination unter Inertgas (z.B. N$_2$), einem Gemisch aus Inertgas und Sauerstoff (z.B. Luft), reduzierend wirkenden Gasen wie Kohlenwasserstoffen (z.B. Methan), Aldehyden (z.B. Acrolein) oder Ammoniak aber auch unter einem Gemisch aus O$_2$ und reduzierend wirkenden Gasen (z.B. allen vorgenannten) erfolgen kann, wie es beispielsweise in der DE-A 4 335 973 (ältere Anmeldung O.Z. 0050/44403) beschrieben wird. Bei einer Calcination unter reduzierenden Bedingungen ist zu beachten, daß die metallischen Konstituenten nicht bis zum Element reduziert werden. Die Calcinationsdauer erstreckt sich in der Regel über einige Stunden und nimmt üblicherweise mit zunehmender Calcinierungstemperatur ab. Wesentlich für die Quellen der elementaren Konstituenten der Multimetalloxidmasse A ist dabei, wie allgemein bekannt, nur, daß es sich entweder bereits um Oxide handelt oder um solche Verbindungen, die durch Erhitzen, wenigstens in Anwesenheit von Sauerstoff, in Oxide überführbar sind. Neben den Oxiden kommen daher als Ausgangsverbindungen vor allem Halogenide, Nitrate, Formiate, Oxalate, Acetate, Carbonate oder Hydroxide in Betracht. Geeignete Ausgangsverbindungen des Mo, V, W und Nb sind auch deren Oxoverbindungen (Molybdate, Vanadate, Wolframate und Niobate) bzw. die von diesen abgeleiteten Säuren.

Multimetalloxidmassen B können in einfacher, dem Fachmann an sich bekannter Weise z.B. dadurch hergestellt werden, daß man von geeigneten Quellen ihrer elementaren Konstituenten ein möglichst inniges, vorzugsweise feinteiliges, Trockengemisch erzeugt und dieses bei Temperaturen von 200 bis 1000°C, vorzugsweise 250 bis 600°C, besonders bevorzugt 300 bis 500°C, mehrere Stunden calciniert, wobei bezüglich der Calcinationsdauer, Calcinationsatmosphäre und Elementquellen das obengenannte gilt. Dabei können hier die dort genannten Calcinationsatmosphären zusätzlich Wasserdampf umfassen.

Das innige Vermischen der Ausgangsverbindungen im Rahmen der Herstellung von Multimetalloxidmassen B kann in trockener oder in nasser Form erfolgen. Erfolgt es in trockener Form, werden die Ausgangsverbindungen zweckmäßigerweise als feinteilige Pulver eingesetzt und nach dem Mischen und gegebenenfalls Verdichten der Calcinierung unterworfen. Vorzugsweise erfolgt das innige Vermischen jedoch in nasser Form. Üblicherweise werden die Ausgangsverbindungen dabei in Form einer wäßrigen Lösung und/oder Suspension miteinander vermischt. Anschließend wird die wäßrige Masse getrocknet und nach Trocknung calciniert. Vorzugsweise erfolgt der Trocknungsprozeß unmittelbar im Anschluß an die Fertigstellung der wäßrigen Mischung und durch Sprühtrocknung (die Austrittstemperaturen betragen in der Regel 100 bis 150°C), die ein besonders inniges Trockengemisch bedingt.

Es überrascht, daß beim ebenda beschriebenen Trockenverfahren, insbesondere dann, wenn die stöchiometrische Zusammensetzung der elementaren Konstituenten derjenigen der allgemeinen Formel III entspricht und das Element Wolfram umfaßt, Kristallite B* erwachsen.

Besonders innige Trockengemische werden beim beschriebenen Trockenverfahren dann erhalten, wenn ausschließlich von in gelöster Form befindlichen Quellen der elementaren Konstituenten ausgegangen wird.

Im Fall des elementaren Konstituenten Kupfer ist es in diesem Zusammenhang besonders vorteilhaft von wäßrigen Lösungen auszugehen, die ihn in Gestalt von Kupfer-Ammoniak (z.B. Tetramin) Komplexen gelöst enthalten.

Bemerkenswerterweise erwächst im Rahmen der Calcinierung solcher besonders inniger Trockengemische, insbesondere wenn diese den elementaren Konstituenten Wolfram umfassen und in ihrer Stöchiometrie der elementaren Konstituenten der allgemeinen Formel III genügen, ein erhöhter Anteil an Kristalliten B*.

In einer bevorzugten Herstellvariante der Multimetalloxidmassen B erfolgt die thermische Behandlung des innigen Gemisches der verwendeten Ausgangsverbindungen in einem Überdruckgefäß (Autoklav) im Beisein von überatmosphärischen Druck aufweisendem Wasserdampf bei Temperaturen im Bereich von > 100 bis 600°C. Der Druckbereich erstreckt sich in typischer Weise auf bis zu 500 atm, vorzugsweise auf bis zu 250 atm. Selbstverständlich können auch Temperaturen oberhalb von 600°C und Drucke oberhalb von 500 atm angewendet werden, was anwendungstechnisch jedoch wenig zweckmäßig ist. Mit besonderem Vorteil erfolgt diese hydrothermale Behandlung unter solchen Bedingungen, unter denen Wasserdampf und flüssiges Wasser koexistieren. Dies ist im Temperaturbereich von > 100°C bis 374,15°C (kritische Temperatur des Wassers) unter Anwendung der entsprechenden Drucke möglich. Die Mengen an Wasser werden dabei zweckmäßig so bemessen, daß die flüssige Phase die Gesamtmenge der Ausgangsverbindungen in Suspension und/oder Lösung aufzunehmen vermag. Möglich ist aber auch eine solche Verfahrensweise, bei der das innige Gemisch der Ausgangsverbindungen die mit dem Wasserdampf im Gleichgewicht befindliche flüssige Wassermenge vollständig absorbiert. Mit Vorteil wird während der hydrothermalen Behandlung gerührt. Zur hydrothermalen Herstellvariante kommen als Ausgangsverbindungen insbesondere alle diejenigen in Betracht, die beim Erhitzen unter Überdruck mit Wasser Oxide und/oder Hydroxide zu bilden vermögen. Vorzugsweise werden als Ausgangsverbindungen bereits Oxide und/oder Hydroxide der elementaren Konstituenten eingesetzt, wobei es besonders günstig ist, von den Elementoxiden auszugehen. In der Regel wird man sie in feinteiliger Form einsetzen.

Das Ergebnis der hydrothermalen Variante umfaßt in der Regel, im Vergleich zur Herstellung durch Calcination eines aus Quellen der elementaren Konstituenten bestehenden innigen Trockengemisches, einen erhöhten Anteil an Kristalliten B*.

Wählt man im Rahmen des hydrothermalen Herstellungsweges die stöchiometrische Zusammensetzung der elementaren Konstituenten gemäß der allgemeinen Formel III, erwachsen in der Regel Kristallite B* mit Vorteil. Häufig werden ausschließlich Kristallite B* erhalten.

Es überrascht; daß bei der hydrothermalen Herstellungsweise auch für von $CuMoO_4$ verschiedene Stöchiometrien der allgemeinen Formel III in der Regel Kristallite B* erwachsen.

Die hydrothermale Behandlung nimmt in typischer Weise mehrere Stunden in Anspruch. Nach beendeter hydrothermaler Behandlung kann dem Autoklaven das in Wasser unlösliche Multimetalloxid B entnommen und nach Trocknung in eine feinteilige Ausgangsmasse 1 überführt werden.

Das innige Inkontaktbringen der Ausgangsmasse 1 mit den Quellen der Multimetalloxidmasse A (Ausgangsmasse 2) kann sowohl trocken als auch naß erfolgen. Im letzteren Fall muß lediglich darauf geachtet werden, daß die vorgebildete Multimetalloxidmasse B nicht in Lösung geht. In wäßrigem Medium ist letzteres bei nicht zu extremen pH-Werten üblicherweise gewährleistet. Erfolgt das innige in Kontakt bringen naß, wird anschließend normalerweise zu einer Trockenmasse getrocknet (vorzugsweise Sprühtrocknen). Eine solche Trockenmasse fällt im Rahmen eines trockenen Mischens automatisch an.

Als mögliche Mischungsvarianten kommen damit z.B. in Betracht:

a) ein trockenes, feinteiliges, vorgebildetes Multimetalloxid B mit trockenen, feinteiligen Ausgangsverbindungen der elementaren Konstituenten des gewünschten Multimetalloxids A im gewünschten Mengenverhältnis in einem Mischer, Kneter oder in einer Mühle mischen;

b) ein feinteiliges Multimetalloxid A vorbilden durch inniges Mischen geeigneter Ausgangsverbindungen ihrer elementaren Konstituenten (trocken o. naß) und anschließendes Calcinieren der daraus resultierenden innigen Trockenmischung bei Temperaturen von 250 bis 450°C (bezüglich Calcinationsdauer, Calcinationsatmosphäre und Elementquellen gilt das auf Seite 8 gesagte); das vorgebildete Multimetalloxid A feinteilig gestalten und mit dem feinteiligen vorgebildeten Multimetalloxid B im gewünschten Mengenverhältnis wie in a) mischen; bei dieser Mischungsvariante ist ein abschließendes Calcinieren der resultierenden Mischung nicht essentiell;

c) in eine wäßrige Lösung und/oder Suspension von Ausgangsverbindungen der elementaren Konstituenten des gewünschten Multimetalloxids A die erforderliche Menge des vorgebildeten Multimetalloxids B einrühren und anschließend sprühtrocknen; selbstverständlich kann anstelle der Ausgangsverbindungen der elementaren Konstituenten des gewünschten Multimetalloxids A auch ein bereits gemäß b) vorgebildetes Multimetalloxid A selbst eingesetzt werden.

Natürlich können auch alle zwischen a), b) und/oder c) liegenden Mischungsvarianten angewendet werden. Das resultierende innige Trockengemisch kann anschließend wie beschrieben calciniert und danach zur gewünschten Katalysatorgeometrie geformt werden oder umgekehrt. Prinzipiell kann das calcinierte (oder bei Anwendung von Mischungsvariante b) gegebenenfalls uncalcinierte) Trockengemisch aber auch als Pulverkatalysator eingesetzt werden.

Eigene Untersuchungen haben ergeben, daß beim Calcinieren des die Ausgangsmasse 1 und die Ausgangsmasse 2 umfassenden Trockengemisches die vorgebildete Multimetalloxidmasse B entweder als solche erhalten bleibt (dies ist insbesondere im Fall eines Oxometallates B* der Fall) oder teilweise oder vollständig in andere Oxometallate B umgewandelt wird. Ein Verschmelzen der Bestandteile der Ausgangsmasse 1 mit jenen der Ausgangsmasse 2 findet jedoch im wesentlichen nicht statt.

Dies eröffnet die Möglichkeit nach Mahlen des vorgebildeten Multimetalloxids B (z.B. durch Naß- oder Trockenmahlen, z.B. in der Kugelmühle oder durch Strahlmahlen) aus dem dabei erhältlichen, in der Regel aus im wesentlichen kugelförmigen Partikeln bestehenden Pulver, die Kornklasse mit einem im für die Masse I gewünschten Größtdurchmesserbereich liegenden Korngrößtdurchmesser (in der Regel > 0 bis 300 μm, vorzugsweise 0,05 bis 200 μm, besonders bevorzugt 0,1 bis 50 μm und ganz besonders bevorzugt 0,1 bis 30 μm) durch in an sich bekannter Weise durchzuführendes Klassieren (z.B. Naß- oder Trockensiebung) abzutrennen und so zur Herstellung der gewünschten Multimetalloxidmasse maßgeschneidert einzusetzen.

Bei Verwendung der erfindungsgemäßen Multimetalloxidmassen als Katalysatoren für die gasphasenkatalytische Oxidation von Acrolein zu Acrylsäure erfolgt die Formgebung zur gewünschten Katalysatorgeometrie vorzugsweise durch Aufbringen auf vorgeformte inerte Katalysatorträger, wobei das Aufbringen vor oder nach der abschließenden Calcination erfolgen kann. Dabei können die üblichen Trägermaterialien wie poröse oder unporöse Aluminiumoxide, Siliciumdioxid, Thoriumdioxid, Zirkondioxid, Siliciumcarbid oder Silicate wie Magnesium- oder Aluminiumsilicat verwendet werden. Die Trägerkörper können regelmäßig oder unregelmäßig geformt sein, wobei regelmäßig geformte Trägerkörper mit deutlich ausgebildeter Oberflächenrauhigkeit, z.B. Kugeln oder Hohlzylinder, bevorzugt werden. Unter diesen sind wiederum Kugeln besonders vorteilhaft. Von besonderem Vorteil ist die Verwendung von im wesentlichen unporösen, oberflächenrauhen, kugelförmigen Trägern aus Steatit, deren Durchmesser 1 bis 6 mm, bevorzugt 4 bis 5 mm beträgt. Die Schichtdicke der Aktivmasse wird zweckmäßigerweise als im Bereich 50 bis 500 μm, bevorzugt im Bereich 150 bis 250 μm liegend, gewählt. Es sei an dieser Stelle darauf hingewiesen, daß bei der Herstellung solcher Schalenkatalysatoren zur Beschichtung der Trägerkörper die aufzubringende Pulvermasse in der Regel befeuchtet und nach dem Aufbringen, z.B. mittels heißer Luft, wieder getrocknet wird.

Die Beschichtung der Trägerkörper wird zur Herstellung der Schalenkatalysatoren in der Regel in einem geeigneten drehbaren Behälter ausgeführt, wie er z.B. aus der DE-A 2909671 oder aus der EP-A 293859 vorbekannt ist. In der Regel wird die relevante Masse vor der Trägerbeschichtung calciniert.

In geeigneter Weise kann das Beschichtungs- und Calcinierungsverfahren gemäß der EP-A 293 859 in an sich bekannter Weise so angewendet werden, daß die resultierenden Multimetalloxidaktivmassen eine spezifische Oberfläche von 0,50 bis 150 m$^2$/g, ein spezifisches Porenvolumen von 0,10 bis 0,90 cm$^3$/g und eine solche Porendurchmesser-Verteilung aufweisen, daß auf die Durchmesserbereiche 0,1 bis < 1 μm, 1,0 bis < 10 μm und 10 μm bis 100 μm jeweils wenigstens 10 % des Porengesamtvolumens entfallen. Vorzugsweise werden die in der EP-A 293 859 als bevorzugt genannten Porendurchmesser-Verteilungen eingestellt.

Selbstverständlich können die erfindungsgemäßen Multimetalloxidmassen auch als Vollkatalysatoren betrieben werden. Diesbezüglich wird das die Ausgangsmasse 1 und 2 umfassende innige Trockengemisch vorzugsweise unmittelbar zur gewünschten Katalysatorgeometrie verdichtet (z.B. Tablettieren, Extrudieren oder Strangpressen), wobei gegebenenfalls an sich übliche Hilfsmittel, z.B. Graphit oder Stearinsäure als Gleitmittel und/oder Formhilfsmittel und Verstärkungsmittel wie Mikrofasern aus Glas, Asbest, Siliciumcarbid oder Kaliumtitanat zugesetzt werden können, und calciniert. Generell kann auch hier vor der Formgebung calciniert werden. Bevorzugte Vollkatalysatorgeometrie sind Hohlzylinder mit einem Außendurchmesser und einer Länge von 2 bis 10 mm und einer Wandstärke von 1 bis 3 mm.

Die erfindungsgemäßen Multimetalloxidmassen eignen sich insbesondere als Katalysatoren mit erhöhter Selektivität (bei vorgegebenem Umsatz) für die gasphasenkatalytische Oxidation von Acrolein zu Acrylsäure. Normalerweise wird bei dem Verfahren Acrolein eingesetzt, das durch die katalytische Gasphasenoxidation von Propen erzeugt wurde. In der Regel werden die Acrolein enthaltenden Reaktionsgase dieser Propenoxidation ohne Zwischenreinigung eingesetzt. Üblicherweise wird die gasphasenkatalytische Oxidation des Acroleins in Rohrbündelreaktoren als heterogene Festbettoxidation ausgeführt. Als Oxidationsmittel wird in an sich bekannter Weise Sauerstoff, zweckmäßigerweise mit inerten Gasen verdünnt, eingesetzt. Geeignete Verdünnungsgase sind z.B. N$_2$, CO$_2$, Kohlenwasserstoff, rückgeführte Reaktionsabgase und/oder Wasserdampf. In der Regel wird bei der Acroleinoxidation ein Acrolein:Sauerstoff:Wasserdampf:Inertgas-Volumenverhältnis von 1:(1 bis 3):(0 bis 20):(3 bis 30) vorzugsweise von 1:(1 bis 3):(0,5 bis 10):(7 bis 18) eingestellt. Der Reaktionsdruck beträgt im allgemeinen 1 bis 3 bar und die Gesamtraumbelastung beträgt vorzugsweise 1.000 bis 3.500 Nl/l/h. Typische Vielrohr-Festbettreaktoren sind z.B. in den Schriften DE-A 2830765, DE-A 2 201 528 oder US-A 3 147 084 beschrieben. Die Reaktionstemperatur wird üblicherweise so gewählt, daß der Acroleinumsatz bei einfachem Durchgang oberhalb von 90 %, vorzugsweise oberhalb von 98 %, liegt. Im Normalfall sind diesbezüglich Reaktionstemperaturen von 230 bis 330°C erforderlich.

Bemerkenswerterweise weisen die erfindungsgemäßen Multimetalloxidmassen im Rahmen der gasphasenkatalytischen Oxidation von Acrolein zu Acrylsäure bezüglich der Selektivität der Acrylsäurebildung auch eine reduzierte Formierungszeit auf; d.h. wird ein mit den erfindungsgemäßen Multimetalloxidmassen beschickter Rohrbündelreaktor unter den vorgenannten Bedingungen mit einem Acrolein enthaltenden Gasstrom zum Zwecke der oxidativen Bildung von Acrylsäure betrieben, so erreicht die Selektivität der Acrylsäurebildung bereits innerhalb einer reduzierten Betriebsdauer ihren Plateauwert. Bezüglich dieses Plateauwertes verfügt die Herstellung der erfindungsgemäßen Multimetalloxidmassen über eine erhöhte Reproduzierbarkeit.

Neben der gasphasenkatalytischen Oxidation von Acrolein zu Acrylsäure vermögen die erfindungsgemäßen Verfahrensprodukte aber auch die gasphasenkatalytische Oxidation anderer organischer Verbindungen wie insbesondere anderer, vorzugsweise 3 bis 6 C-Atome aufweisender, Alkane, Alkanole, Alkanale, Alkene und Alkenole (z.B. Propylen, Methacrolein, tert.-Butanol, Methylether des tert.-Butanol, iso-Buten, iso-Butan oder iso-Butyraldehyd) zu olefinisch ungesättigten Aldehyden und/oder Carbonsäuren, sowie den entsprechenden Nitrilen (Ammoxidation, vor allem von Propen zu Acrylnitril und von iso-Buten bzw. tert.-Butanol zu Methacrylnitril) zu katalysieren. Beispielhaft genannt sei die Herstellung von Acrolein, Methacrolein und Methacrylsäure. Sie eignen sich aber auch zur oxidativen Dehydrierung olefinischer Verbindungen.

Umsatz, Selektivität und Verweilzeit sind in dieser Schrift, falls nichts anderes erwähnt wird, wie folgt definiert:

$$\text{Umsatz U an Acrolein (\%)} = \frac{\text{Molzahl umgesetztes Acrolein}}{\text{Molzahl eingesetztes Acrolein}} \times 100;$$

$$\text{Selektivität S der Acrylsäurebildung \%} = \frac{\text{Molzahl Acrolein umgesetzt zu Acrylsäure}}{\text{Molzahl Acrolein insgesamt umgesetzt}} \times 100;$$

$$\text{Verweilzeit (sec)} = \frac{\text{mit Katalysator gefülltes Leervolumen des Reaktors (l)}}{\text{durchgesetzte Synthesegasmenge (Nl/h)}} \times 3.600;$$

Beispiele

a) Herstellung von erfindungsgemäßen Multimetalloxidmassen M und Multimetalloxidmassen MV zum Vergleich

MV1: 127 g Kupfer(II)acetatmonohydrat wurden in 2.700 g Wasser zu einer Lösung I gelöst. In 5.500 g Wasser wurden bei 95°C nacheinander 860 g Ammoniumheptamolybdattetrahydrat, 143 g Ammoniummetavanadat und 126 g Ammoniumparawolframatheptahydrat zu einer Lösung II gelöst. Anschließend wurde die Lösung I auf einmal in die Lösung II eingerührt und das wäßrige Gemisch bei einer Austrittstemperatur von 110°C sprühgetrocknet. Danach wurde das Sprühpulver je kg Pulver mit 0,15 kg Wasser verknetet. Die Knetmasse wurde in einem mit einem Sauerstoff/Stickstoff-Gemisch beschickten Umluftofen calciniert. Der Sauerstoffgehalt wurde dabei so eingestellt, daß am Ausgang des Umluftofens ein $O_2$-Gehalt von 1,5 Vol.-% bestand. Im Rahmen der Calcinierung wurde die Knetmasse zunächst mit einer Geschwindigkeit von 10 K/min auf 300°C aufgeheizt und anschließend während 6 h auf dieser Temperatur gehalten. Danach wurde mit einer Geschwindigkeit von 10 K/min auf 400°C aufgeheizt und diese Temperatur noch 1 h aufrechterhalten. Zur Einstellung des Ammoniakgehaltes der Calcinierungsatmosphäre wurden die Ofenbeladung O (g Katalysatorvorläufer pro l Innenvolumen des Umluftofens), der Eingangsvolumenstrom ES (Nl/h) des Sauerstoff/Stickstoff-Gemisches und die Verweilzeit VZ (sec) der Sauerstoff/Stickstoff-Beschickung (Verhältnis aus Innenvolumen des Umluftofens und Volumenstrom des zugeführten Sauerstoff/Stickstoff-Gemisches) wie nachfolgend aufgelistet gewählt. Der verwendete Umluftofen wies ein Innenvolumen von 3 l auf.

O: 250 g/l,
VZ: 135 sec und
ES: 80 Nl/h.

Dem resultierenden katalytisch aktiven Material liegt folgende Stöchiometrie zugrunde:
$Mo_{12}V_3W_{1,2}Cu_{1,6}O_x$.
Nach Mahlen des calcinierten, katalytisch aktiven Materials auf Teilchendurchmesser im Bereich von 0,1 bis 50 μm wurden mit dem dabei resultierenden Aktivmassenpulver in einer Drehtrommel unporöse, oberflächenrauhe Steatitkugeln eines Durchmessers von 4 bis 5 mm in einer Menge von 50 g Pulver je 200 g Steatitkugeln bei gleichzeitigem Zusatz von 18 g Wasser beschichtet. Anschließend wurde mit 110°C heißer Luft getrocknet.

M1:

Ausgangsmasse 1:

Gemäß A. Moini et al., Inorg. Chem. 25 (21) (1986) S. 3782 bis 3785, insbesondere S. 3782 bis S. 3783, wurde $Cu_4Mo_6O_{20}$ in feinteiliger Form hergestellt (zahlenmittlerer Korndurchmesser $\overline{d}$ = 8 µm).

Ausgangsmasse 2:

Wäßrige Lösung von Ammoniumheptamolybdattetrahydrat, Ammoniummetavanadat und Ammoniumparawolframatheptahydrat in solchen Mengen, daß der wäßrigen Lösung nachfolgende Elementstöchiometrie zugrunde lag:

$Mo_{12}V_{3,75}W_{1,5}$.

Von der Ausgangsmasse 1 wurde soviel in die Ausgangsmasse 2 eingerührt, daß das molare Verhältnis der vorgenannten stöchiometrischen Einheiten 0,4 (Ausgangsmasse 1) zu 0,8 (Ausgangsmasse 2) betrug.

Anschließend wurde die wäßrige Mischung wie in MV1 sprühgetrocknet und zu einem Schalenkatalysator weiterverarbeitet.

M2:

Ausgangsmasse 1:

Gemäß E.M. McCarron III und J.C. Calabrese, J. Solid State Chem. 65 (1986) S. 215 bis 224, insbesondere S. 215 bis 216, wurde $Cu_4Mo_5O_{17}$ (Karteikarte 39 - 181 der JCPDS-ICDD Kartei (1991)) in feinteiliger Form hergestellt (zahlenmittlerer Korndurchmesser $\overline{d}$ = 8 µm).

Ausgangsmasse 2:

Wäßrige Lösung wie bei M1, die zugrunde liegende Elementstöchiometrie war jedoch:

$Mo_{12}V_{3,6}W_{1,44}$.

Von der Ausgangsmasse 1 wurde soviel in die Ausgangsmasse 2 eingerührt, daß das molare Verhältnis der vorgenannten stöchiometrischen Einheiten 0,4 (Ausgangsmasse 1) zu 0,83 (Ausgangsmasse 2) betrug.

Anschließend wurde die wäßrige Mischung wie in MV1 sprühgetrocknet und zu einem Schalenkatalysator weiterverarbeitet.

M3:

Ausgangsmasse 1:

Gemäß E.M. McCarron III u. J.C. Calabrese, J. Solid State Chem. 62 (1986) S. 64 bis 74, insbesondere S. 65, wurde $Cu_6Mo_5O_{18}$ (Karteikarte 40 - 865 der JCPDS-ICDD Kartei (1991)) in feinteiliger Form hergestellt (zahlenmittlerer Korndurchmesser $\overline{d}$ = 8 µm).

Ausgangsmasse 2:

Wäßrige Lösung wie bei M1, die zugrunde liegende Elementstöchiometrie war jedoch:

$Mo_{12}V_{3,37}W_{1,35}$.

Von der Ausgangsmasse 1 wurde soviel in die Ausgangsmasse 2 eingerührt, daß das molare Verhältnis der vorgenannten stöchiometrischen Einheiten 0,27 (Ausgangsmasse 1) zu 0,89 (Ausgangsmasse 2) betrug.

Anschließend wurde die wäßrige Mischung wie in MV1 sprühgetrocknet und zu einem Schalenkatalysator weiterverarbeitet.

M4:

Ausgangsmasse 1:

Gemäß K. Nassau u. J.W. Shiever, J. Am. Ceram. Soc. 52 (1) (1969) S. 36 bis 40, insbesondere S. 36, wurde $CuMoO_4$ (Karteikarte 22 - 242 der JCPDS-ICDD-Kartei (1991)) in feinteiliger Form hergestellt (zahlenmittlerer Korndurchmesser $\overline{d}$ = 8 µm).

Ausgangsmasse 2:

Wäßrige Lösung wie bei M1, die zugrundeliegende Elementstöchiometrie war jedoch:

$Mo_{12}V_{3,46}W_{1,38}$.

Von der Ausgangsmasse 1 wurde soviel in die Ausgangsmasse 2 eingerührt, daß das molare Verhältnis der vorgenannten stöchiometrischen Einheiten 1,6 (Ausgangsmasse 1) zu 0,87 (Ausgangsmasse 2) betrug.

Anschließend wurde die wäßrige Mischung wie in MV1 sprühgetrocknet und zu einem Schalenkatalysator weiterverarbeitet.

M5:

Ausgangsmasse 1:

In 500 ml Wasser wurden 55,3 g Cu(II)-Oxid (CuO, Fa. Merck, Darmstadt, reinst, mindestens 96 %, pulverförmig) und 100,0 g Mo(VI)-Oxid ($MoO_3$, Fa. Merck, Darmstadt, p.a., mindestens 99,5 %, pulverförmig) eindispergiert. Die Gesamtmenge der wäßrigen Dispersion wurde in einem Autoklaven (Werkstoff: Hastelloy C4; Innenvolumen: 2,5 l) unter Rühren (1000 Umdrehungen pro Minute) auf 350°C erwärmt und bei dieser Temperatur und dem zugehörigen Überdruck während 24 h unter Rühren gehalten. Anschließend wurde der Autoklav auf Raumtemperatur abgekühlt, die darin enthaltene wäßrige Dispersion entnommen, der dispergierte Feststoff abfiltriert und anschließend im Trockenschrank bei 80°C getrocknet. Das resultierende trokkene Pulver wies bei der rasterelektronenmikroskopischen Untersuchung (REM) kristalline Partikel mit einem zahlenmittleren Korngrößendurchmesser von etwa 8 µm aus. Die chemische Analyse der kristallinen Partikel ergab ein Cu/Mo-Verhältnis von ca. 1.

Unter Anwendung von Cu-K$\alpha$-Strahlung (Siemens-Diffraktometer D-5000, 40 kV, 30 mA, mit automatischer Divergenz-, Streustrahl- und Zählrohrblende und Peltier-Detektor) zeigte das kristalline Pulver CuMoOy das nachfolgende Röntgenbeugungsmuster, wiedergegeben in Gestalt von von der Wellenlänge der verwendeten Röntgenstrahlung unabhängigen Netzebenenabständen d[Å], sowie den zugehörigen, auf die intensitätsstärkste Beugungslinie bezogenen, relativen Intensitäten (%) der verschiedenen Beugungslinien:

| d [Å] | Intensität [%] |
|-------|----------------|
| 2,44  | 100            |
| 3,01  | 58,4           |
| 3,14  | 56,8           |
| 2,75  | 35,5           |
| 2,82  | 30,6           |
| 3,39  | 30,1           |
| 1,65  | 25,2           |
| 3,96  | 21,6           |
| 1,72  | 21,1           |
| 2,50  | 20,5           |
| 2,20  | 17,3           |
| 4,68  | 15,2           |
| 2,48  | 14,5           |
| 1,96  | 13,8           |
| 3,71  | 13,7           |
| 3,75  | 13,2           |
| 1,80  | 12,4           |
| 2,90  | 12,2           |
| 2,34  | 12,1           |
| 1,61  | 11,8           |
| 1,59  | 11,6           |
| 3,31  | 11,5           |
| 1,85  | 11,5           |
| 2,04  | 11,3           |
| 2,08  | 11,2           |
| 1,70  | 11,1           |
| 2,00  | 10,8           |
| 1,89  | 10,7           |
| 2,12  | 10,3           |

EP 0 668 104 B1

| d [Å] | Intensität [%] |
|---|---|
| 1,88 | 9,15 |
| 1,86 | 8,52 |
| 1,98 | 8,25 |
| 2,30 | 8,01 |
| 2,04 | 7,29 |
| 2,66 | 6,89 |
| 1,57 | 6,73 |
| 1,55 | 6,54 |
| 1,77 | 6,53 |
| 2,37 | 6,45 |
| 1,56 | 6,03 |
| 1,55 | 5,93 |
| 3,45 | 5,82 |
| 2,12 | 5,79 |
| 1,63 | 5,76 |
| 2,06 | 5,72 |
| 1,83 | 5,43 |
| 1,60 | 5,42 |
| 2,14 | 5,12 |
| 5,81 | 4,91 |

Die Ungenauigkeit der Angabe der Netzebenenabstände d beläuft sich auf ± 0,20 Å (die intensitätsarmen Linien umfassen vermutlich auch auf geringfügige Verunreinigungen zurückgehende Linien). Dieses Röntgenbeugungsmuster entspricht demjenigen für $CuMoO_4$-III in Russian Journal of Inorganic Chemistry 36 (7), 1991, S. 927, Table 1.

Ausgangsmasse 2:

Ein feinteiliges Trockengemisch von Ammoniumheptamolybdattetrahydrat, Ammoniummetavanadat und Ammoniumparawolframatheptahydrat, der nachfolgende Elementstöchiometrie zugrunde lag: $Mo_{12}V_{3,46}W_{1,38}$.

Von der Ausgangsmasse 1 wurde soviel in die Ausgangsmasse 2 eingerührt, daß das molare Verhältnis der vorgenannten stöchiometrischen Einheiten im resultierenden Trockengemisch 1,6 (Ausgangsmasse 1) zu 0,87 (Ausgangsmasse 2) betrug. Anschließend wurde das Trockengemisch wie das im Rahmen der Sprühtrocknung bei MV1 anfallende Sprühpulver zu einem Schalenkatalysator weiterverarbeitet.

M6:

Ausgangsmasse 1:
Das feinteilige CuMoOy aus M5.
Ausgangsmasse 2:

Das gleiche Gemisch wie bei M5, allerdings in Wasser gelöst.

Von der Ausgangsmasse 1 wurde soviel in die Ausgangsmasse 2 eingerührt, daß das molare Verhältnis der vorgenannten stöchiometrischen Einheiten 1,6 (Ausgangsmasse 1) zu 0,87 (Ausgangsmasse 2) betrug.

Anschließend wurde die wäßrige Mischung wie in MV1 sprühgetrocknet und zu einem Schalenkatalysator weiterverarbeitet. Letzterer enthielt das Röntgenbeugungsmuster der Ausgangsmasse 1.

M7:

Wie M6, die Ausgangsmasse M1 wurde jedoch auf eine zahlenmittlere Körnung von $\bar{d} = 4\ \mu m$ gemahlen.

Auch hier enthielt der resultierende Schalenkatalysator das B* Röntgenbeugungsmuster.

M8:

Wie M6, vor der Sprühtrocknung der wäßrigen Mischung wurde in selbige jedoch zusätzlich Kupfer(II)acetat-monohydrat eingerührt, und zwar, bezogen auf die stöchiometrische Einheit $Mo_{12}V_{3,46}W_{1,38}$ des bereits im wäßrigen Gemisch Gelösten, in einer stöchiometrischen Häufigkeit des Kupfers von 0,8.

Anschließend wurde die wäßrige Mischung wie in MV1 sprühgetrocknet und zu einem Schalenkatalysator weiterverarbeitet, der ebenfalls das B* Röntgenbeugungsmuster enthielt.

MV2: In 1400 ml Wasser wurden 172,7 g Ammoniummolybdat, 43,9 g Ammoniummetavanadat und 6,0 g Ammoniumdichromat gelöst. Davon räumlich getrennt wurde eine zweite Lösung von 43,9 g Kupfernitrat in 75 ml Wasser hergestellt, das mit 3 ml konzentrierter Salpetersäure angesäuert worden war. Anschließend wurde die zweite Lösung der ersten Lösung unter Rühren und Erwärmen tropfenweise zugesetzt. Anschließend wurde das wäßrige Gemisch wie in MV1 sprühgetrocknet und zu einem Schalenkatalysator weiterverarbeitet. Dem resultierenden katalytisch aktiven Material liegt folgende Stöchiometrie zugrunde: $Mo_{12}V_{4,6}Cr_{0,56}Cu_{2,22}O_x$.

M9:

Ausgangsmasse 1:
Das feinteilige CuMoOy aus M5.
Ausgangsmasse 2:
Ammoniummolybdat, Ammoniummetavanadat und Ammoniumdichromat wurden im stöchiometrischen Verhältnis $Mo_{12}V_{5,6}Cr_{0,69}$ in Wasser gelöst.
Von der Ausgangsmasse 1 wurde soviel in die Ausgangsmasse 2 eingerührt, daß das molare Verhältnis der vorgenannten stöchiometrischen Einheiten 2,22 (Ausgangsmasse 1) zu 0,815 (Ausgangsmasse 2) betrug.
Anschließend wurde die wäßrige Mischung wie in MV1 sprühgetrocknet und zu einem Schalenkatalysator weiterverarbeitet. Letzterer enthielt das Röntgenbeugungsmuster gemäß B*.

M10:

Ausgangsmasse 1:
In 500 ml Wasser wurden 55,3 g Cu(II)-Oxid (CuO, Fa. Merck, Darmstadt, reinst, mindestens 96 %, pulverförmig), 70,1 g Mo(VI)-Oxid ($MoO_3$, Fa. Merck, Darmstadt, p.a., mindestens 99,5 %), 11,4 g V(V)-Oxid ($V_2O_5$, Fa. Merck, Darmstadt, reinst, mindestens 99 %) und 20,9 g Wolframsäure ($H_2WO_4$, Fa. Merck, Darmstadt, reinst, mindestens 98 %) eindispergiert.
Die resultierende wäßrige Dispersion wurde entsprechend der Herstellung der Ausgangsmasse 1 in M5 behandelt.
Es wurde ein im wesentlichen kristallines Pulver der Stöchiometrie $Cu_{50}Mo_{35}V_9W_6O_y$ erhalten, das ein zur Ausgangsmasse 1 aus M5 analoges Röntgenbeugungsmuster aufwies. Der zahlenmittlere Korndurchmesser betrug ca. 8 μm.
Ausgangsmasse 2:
Ammoniummolybdat, Ammoniummetavanadat und Ammoniumparawolframat wurden im stöchio-

metrischen Verhältnis $Mo_{12}V_3W_{1,11}$ in Wasser gelöst.

Von der Ausgangsmasse 1 wurde soviel in die Ausgangsmasse 2 eingerührt, daß das molare Verhältnis der vorgenannten stöchiometrischen Einheiten 0,91 (Ausgangsmasse 2) zu 0,032 (Ausgangsmasse 1) betrug.

Anschließend wurde die wäßrige Mischung wie in MV1 sprühgetrocknet und zu einem Schalenkatalysator weiterverarbeitet. Dieser zeigte auch das B* Röntgenbeugungsmuster.

MV3: Wie M1, als Ausgangsmasse 1 wurde jedoch $Bi_2 Mo_3 O_{12}$ ($\hat{=} Bi_4 Mo_6 O_{24}$) gemäß J. Hinz, Gmelin Mo Erg.-Bd. B1, S. 146 bis 157, insbesondere S. 151 bis 152, in entsprechend feinteiliger Form verwendet.

Ferner wurde (aus Gründen der Stöchiometrie) von der Ausgangsmasse 1 soviel in die Ausgangsmasse 2 eingerührt, daß das molare Verhältnis der stöchiometrischen Einheiten 0,8 (Ausgangsmasse 1) zu 0,8 (Ausgangsmasse 2) betrug.

MV4: Wie M4, als Ausgangsmasse 1 wurde jedoch $Bi_2 Mo_2 O_9$ ($\hat{=} Bi_4 Mo_4 O_{18}$) gemäß J. Hinz, Gmelin Mo Erg.-Bd. B1, S. 146 bis 157, insbesondere S. 152 bis 153, in entsprechend feinteiliger Form verwendet.

Ferner wurde (aus Gründen der Stöchiometrie) von der Ausgangsmasse 1 soviel in die Ausgangsmasse 2 eingerührt, daß das molare Verhältnis der stöchiometrischen Einheiten 0,8 (Ausgangsmasse 1) zu 0,87 (Ausgangsmasse 2) betrug.

M11:

Ausgangsmasse 1:

Gemäß T. Machej und J. Ziolkowski, Bull. Acad. Pol. Sci., Ser. Sci. Chim. 24 (1976) 425-431, wurde $Cu_3Mo_2O_9$ in feinteiliger Form hergestellt (zahlenmittlerer Korndurchmesser $\bar{d}$ = 8 µm).

Ausgangsmasse 2:

Wäßrige Lösung wie bei M1, die zugrunde liegende Elementstöchiometrie war jedoch:

$Mo_{12} V_{3,29} W_{1,32}$.

Von der Ausgangsmasse 1 wurde soviel in die Ausgangsmasse 2 eingerührt, daß das molare Verhältnis der vorgenannten stöchiometrischen Einheiten 0,53 (Ausgangsmasse 1) zu 0,91 (Ausgangsmasse 2) betrug.

Anschließend wurde die wäßrige Mischung wie in MV1 sprühgetrocknet und zu einem Schalenkatalysator weiterverarbeitet.

M12:

Ausgangsmasse 1:

Zur Herstellung von $Cu Mo_{0,74} V_{0,14} W_{0,12} O_{3,93}$ wurden zunächst zwei wäßrige Gemische G1 und G2 hergestellt.

G1: Bei 25°C wurden 373 g $Cu(CH_3COO)_2 \cdot H_2O$ (Cu Gehalt : 32,5 Gew.-%) und 165 g 25 gew.-%ige wäßrige $NH_3$-Lösung nacheinander in 3 l Wasser eingerührt und das resultierende Gemisch anschließend bei 25°C noch 1 h gerührt.

G2: 248 g $(NH_4)_6 Mo_7 O_{24} \cdot 4H_2O$ ($MoO_3$-Gehalt: 81,3 Gew.-%), 31 g $NH_4VO_3$ ($V_2O_5$-Gehalt: 76,8 Gew.-%) und 62 g $(NH_4)_{10} W_{12} O_{41} \cdot 7H_2O$ ($WO_3$-Gehalt: 89,2 Gew.-%) wurden nacheinander in 5 l Wasser bei 90°C unter Rühren gelöst.

Anschließend wurde G1 in G2 eingerührt und das dabei resultierende wäßrige Gemisch G3 noch 1 h unter Rühren bei 80°C gehalten. Danach wurde G3 bei einer Eingangstemperatur von 310°C und einer Ausgangstemperatur von 110°C sprühgetrocknet. Das resultierende Sprühpulver wurde in 200 g Portionen in flachen Porzellanschalen innerhalb von 6 h an der Luft von 25°C auf 220°C erwärmt (lineare Aufheizrate) und dann 12 h bei dieser Temperatur an der Luft belassen. Anschließend wurde das bei 220°C vorgetemperte Produkt noch 1 h an der Luft bei 400°C getempert und danach auf 25°C abgekühlt.

Von dem erhaltenen Pulver der oben genannten Stöchiometrie wurde unter Anwendung von Cu-$K_\alpha$-Strahlung eine Röntgenaufnahme (Pulver-Röntgendiffraktogramm) aufgenommen. Durch Vergleich mit bekannten Röntgendiagrammen bekannter Substanzen, konnte das Pulver-Röntgendiffraktogramm auf nachfolgende Phasenzusammensetzung abgebildet werden:

ca. 65 Gew.-% eines mit V und W dotierten Kupfermolybdats der Struktur des $CuMoO_4$-III

gemäß Russian Journal of Inorganic Chemistry 36 (7), 1991, S. 927, Table 1 (Wolframit-Struktur) und

ca. 35 Gew.-% eines mit V und W dotierten Kupfermolybdats der Struktur des $CuMoO_4$ gemäß Karteikarte 22-242 der JCPDS-ICDD Kartei (1991).

Ausgangsmasse 2:
Wäßrige Lösung wie bei M1, die zugrunde liegende Elementstöchiometrie war jedoch:
$Mo_{12} V_{3,09} W_{1,10}$.
Von der Ausgangsmasse 1 wurde soviel in die Ausgangsmasse 2 eingerührt, daß das molare Verhältnis der vorgenannten stöchiometrischen Einheiten 1,6 (Ausgangsmasse 1) zu 0,90 (Ausgangsmasse 2) betrug.
Anschließend wurde die wäßrige Mischung wie in MV1 sprühgetrocknet und zu einem Schalenkatalysator weiterverarbeitet. Dieser enthielt ebenfalls das Röntgenbeugungsmuster des Wolframit-Typs.

M13:

Ausgangsmasse 1:
Zur Herstellung von $CuMo_{0,75} W_{0,25} O_4$ wurde wie zur Herstellung der Ausgangsmasse 1 in M12 vorgegangen. Die Zusammensetzungen von G1 und G2 waren jedoch:

G1: 456 g $Cu(CH_3COO)_2 \cdot H_2O$ (Cu-Gehalt: 32,5 Gew.-%),
128 g 25 gew.-%ige wäßrige $NH_3$-Lösung und 3 l $H_2O$.
G2: 310 g $(NH_4)_6 Mo_7 O_{24} \cdot 4H_2O$ ($MoO_3$-Gehalt: 81,3 Gew.%),
151 g $(NH_4)_{10} W_{12} O_{41} \cdot 7H_2O$ ($WO_3$-Gehalt: 89,2 Gew.-%) und 5 l $H_2O$.

Außerdem erfolgte die Nachtemperung an Luft nicht 1 h bei 400°C, sondern 1 h bei 500°C.
Von dem erhaltenen Pulver der oben genannten Stöchiometrie wurden unter Anwendung von $Cu-K_\alpha$-Strahlung eine Röntgenaufnahme (Pulver-Röntgendiffraktogramm) aufgenommen. Durch Vergleich mit bekannten Röntgendiagrammen bekannter Substanzen, konnte das Pulver-Röntgendiffraktiogramm auf nachfolgende Phasenzusammensetzung abgebildet werden:

ca. 50 Gew.-% eines mit W dotierten Kupfermolybdats der Struktur des $CuMoO_4$-III gemäß Russian Journal of Chemistry 36 (7), 1991, S. 927, Table 1 (Wolframit-Struktur) und
ca. 50 Gew.-% eines mit W dotierten Kupfermolybdats der Struktur des $CuMoO_4$ gemäß Karteikarte 22-242 der JCPDS-ICDD Kartei (1991).

Ausgangsmasse 2:
Wäßrige Lösung wie bei M1, die zugrunde liegende Elementstöchiometrie war jedoch:
$Mo_{12} V_{3,33} W_{0,89}$.
Von der Ausgangsmasse 1 wurde soviel in die Ausgangsmasse 2 eingerührt, daß das molare Verhältnis der vorgenannten stöchiometrischen Einheiten 1,6 (Ausgangsmasse 1) zu 0,90 (Ausgangsmasse 2) betrug.
Anschließend wurde die wäßrige Mischung wie in MV1 sprühgetrocknet und zu einem Schalenkatalysator weiterverarbeitet. Dieser enthielt ebenfalls das Röntgenbeugungsmuster des Wolframit-Typs.

M14:

Ausgangsmasse 1:
Zur Herstellung von $CuMo_{0,5} W_{0,5} O_4$ wurde wie zur Herstellung der Ausgangsmasse 1 in M12 vorgegangen. Die Zusammensetzungen von G1 und G2 waren jedoch:

G1: 493 g $Cu(CH_3COO)_2 \cdot H_2O$ (Cu-Gehalt: 32,5 Gew.-%),
198 g 25 gew.-%ige wäßrige $NH_3$-Lösung und 3 l $H_2O$.
G2: 223 g $(NH_4)_6 Mo_7 O_{24} \cdot 4H_2O$ ($MoO_3$-Gehalt: 81,3 Gew.-%),
327 g $(NH_4)_{10} W_{12} O_{41} \cdot 7H_2O$ ($WO_3$-Gehalt: 89,2 Gew.-%) und 5 l $H_2O$.

Außerdem erfolgte die Nachtemperung an Luft nicht 1 h bei 400°C, sondern 1 h bei 500°C.

Von dem erhaltenen Pulver der oben genannten Stöchiometrie wurden unter Anwendung von Cu-$K_\alpha$-Strahlung eine Röntgenaufnahme (Pulver-Röntgendiffraktogramm) aufgenommen. Das Pulver erwies sich als einphasig. Es wies zu 100 % Wolframit-Struktur (Strukturtyp des Cu-$MoO_4$-III gemäß Russian Journal of Inorganic Chemistry 36 (7), 1991, S. 927, Table 1) auf.

Ausgangsmasse 2:

Wäßrige Lösung wie bei M1, die zugrunde liegende Elementstöchiometrie war jedoch:

$Mo_{12} V_{3,21} W_{o,43}$.

Von der Ausgangsmasse 1 wurde soviel in die Ausgangsmasse 2 eingerührt, daß das molare Verhältnis der vorgenannten stöchiometrischen Einheiten 1,6 (Ausgangsmasse 1) zu 0,93 (Ausgangsmasse 2) betrug.

Anschließend wurde die wäßrige Mischung wie in MV1 sprühgetrocknet und zu einem Schalenkatalysator weiterverarbeitet. Dieser enthielt ebenfalls das Röntgenbeugungsmuster des Wolframit-Typs.

M15:

Wie M12, bei der Herstellung der Ausgangsmasse 1 wurden jedoch anstelle von 165 g 25 gew.-%iger wäßriger $NH_3$-Lösung 435 g 25 gew.-%ige wäßrige $NH_3$-Lösung zur Herstellung des Gemisches G1 verwendet.

Außerdem wurde das resultierende Sprühpulver in 200 g Portionen in flachen Porzellanschalen innerhalb von 3 h an der Luft von 25°C auf 300°C erwärmt (lineare Aufheizrate) und dann 1 h bei dieser Temperatur an der Luft belassen. Anschließend wurde das bei 300°C vorgetemperte Produkt noch 1 h an der Luft bei 400°C getempert und danach auf 25°C abgekühlt.

Von dem so als Ausgangsmasse 1 erhaltenen Pulver der Stöchiometrie $CuMo_{0,74} V_{0,14} W_{0,12} O_{3,93}$ wurde unter Anwendung von Cu-$K_\alpha$-Strahlung eine Röntgenaufnahme (Pulver-Röntgendiffraktogramm) aufgenommen. Durch Vergleich mit bekannten Röntgendiagrammen bekannter Substanzen ergab sich, daß das Pulver zu > 95 Gew.-% aus einem mit V und W dotierten Kupfermolybdat der Struktur des $CuMoO_4$-III gemäß Russian Journal of Inorganic Chemistry 36(7), 1991, S. 927, Table 1 (Wolframit-Struktur) bestand.

b) Verwendung der Schalenkatalysatoren aus a) als Katalysatoren für die Gasphasenoxidation von Acrolein zu Acrylsäure

Die Katalysatoren wurden in einen Rohrreaktor gefüllt (V2A-Stahl, 25 mm Innendurchmesser, 2000 g Katalysatorschüttung, Salzbadtemperierung) und bei Reaktionstemperaturen im Bereich von 250 bis 270°C unter Anwendung einer Verweilzeit von

2,0 sec mit einem gasförmigen Gemisch der Zusammensetzung
5 Vol.-% Acrolein,
7 Vol.-% Sauerstoff,
15 Vol.-% Wasserdampf und
73 Vol.-% Stickstoff

beschickt. Die Salzbadtemperatur wurde in allen Fällen so eingestellt, daß, nach beendeter Formierung, bei einfachem Durchgang ein einheitlicher Acroleinumsatz U von 99 % resultierte. Das aus dem Rohrreaktor strömende Produktgasgemisch wurde gaschromatographisch analysiert. Die Ergebnisse für die Selektivität der Acrylsäurebildung in Anwendung der verschiedenen Katalysatoren zeigt die nachfolgende Tabelle.

| Katalysator | S (%) |
|---|---|
| MV1 | 95,3 |
| M1 | 95,4 |
| M2 | 95,4 |
| M3 | 95,6 |

(fortgesetzt)

| Katalysator | S (%) |
|---|---|
| M4 | 95,7 |
| M5 | 95,5 |
| M6 | 95,9 |
| M7 | 96,0 |
| M8 | 96,0 |
| M10 | 95,8 |
| MV2 | 93,4 |
| M9 | 93,9 |
| MV3 | 92,6 |
| MV4 | 92,2 |
| M11 | 95,5 |
| M12 | 96,5 |
| M13 | 96,5 |
| M14 | 96,0 |
| M15 | 96,8 |

**Patentansprüche**

1. Multimetalloxidmassen der allgemeinen Formel I

$$[A]_p [B]_q \qquad (I),$$

in der die Variablen folgende Bedeutung haben:

A $\quad Mo_{12} V_a X_b^1 X_c^2 X_d^3 X_e^4 X_f^5 X_g^6 O_x$ (Co-Phase),
B $\quad X_{12}^7 Cu_h H_i O_y$ (Schlüsselphase),
$X^1$ W, Nb, Ta, Cr und/oder Ce,
$X^2$ Cu, Ni, Co, Fe, Mn und/oder Zn,
$X^3$ Sb und/oder Bi,
$X^4$ Li, Na, K, Rb, Cs und/oder H,
$X^5$ Mg, Ca, Sr und/oder Ba,
$X^6$ Si, Al, Ti und/oder Zr,
$X^7$ Mo, W, V, Nb und/oder Ta,
a 1 bis 8,
b 0,2 bis 5,
c 0 bis 23,
d 0 bis 50,
e 0 bis 2,
f 0 bis 5,
g 0 bis 50,
h 4 bis 30,
i 0 bis 20,
x, y Zahlen, die durch die Wertigkeit und Häufigkeit der von Sauerstoff verschiedenen Elemente in I bestimmt werden und
p, q von Null verschiedene Zahlen, deren Verhältnis p/q 160:1 bis 1:1 beträgt,

die den Anteil $[A]_p$ in Form dreidimensional ausgedehnter, von ihrer lokalen Umgebung aufgrund ihrer von ihrer lokalen Umgebung verschiedenen chemischen Zusammensetzung abgegrenzter, Bereiche A der chemischen

**17**

Zusammensetzung

A $\quad Mo_{12} V_a X_b^1 X_c^2 X_d^3 X_e^4 X_f^5 X_g^6 O_x$

und den Anteil $[B]_q$ in Form dreidimensional ausgedehnter, von ihrer lokalen Umgebung aufgrund ihrer von ihrer lokalen Umgebung verschiedenen chemischen Zusammensetzung abgegrenzter, Bereiche B der chemischen Zusammensetzung

B $\quad X_{12}^7 Cu_h H_i O_y$

enthalten, wobei die Bereiche A, B relativ zueinander wie in einem Gemisch aus feinteiligem A und feinteiligem B verteilt sind.

2.  Multimetalloxidmassen nach Anspruch 1, mit $X^1$ = W, Nb und/oder Cr.

3.  Multimetalloxidmassen nach Anspruch 1 oder 2, mit $X^2$ = Cu, Ni, Co und/oder Fe.

4.  Multimetalloxidmassen nach Anspruch 1 bis 3, mit $X^3$ = Sb.

5.  Multimetalloxidmassen nach Anspruch 1 bis 4, mit $X^4$ = Na und/oder K.

6.  Multimetalloxidmassen nach Anspruch 1 bis 5, mit $X^5$ = Ca, Sr und/oder Ba.

7.  Multimetalloxidmassen nach Anspruch 1 bis 6, mit $X^6$ = Si, Al und/oder Ti.

8.  Multimetalloxidmassen nach Anspruch 1 bis 7, mit $X^7$ = Mo.

9.  Multimetalloxidmassen nach Anspruch 1 bis 8, mit a = 3 bis 6.

10.  Multimetalloxidmassen nach Anspruch 1 bis 9 mit b = 0,5 bis 2,5.

11.  Multimetalloxidmassen nach Anspruch 1 bis 10, mit c = 0 bis 4.

12.  Multimetalloxidmassen nach Anspruch 1 bis 11, mit d = 0 bis 3.

13.  Multimetalloxidmassen nach Anspruch 1 bis 12, mit e = 0 bis 0,3.

14.  Multimetalloxidmassen nach Anspruch 1 bis 13, mit f = 0 bis 2.

15.  Multimetalloxidmassen nach Anspruch 1 bis 14, mit g = 0 bis 20.

16.  Multimetalloxidmassen nach Anspruch 1 bis 15, mit h = 6 bis 24.

17.  Multimetalloxidmassen nach Anspruch 1 bis 15, mit h = 9 bis 18.

18.  Multimetalloxidmassen nach Anspruch 1 bis 17, mit p/q = 20:1 bis 1:1.

19.  Multimetalloxidmassen nach Anspruch 1 bis 17, mit p/q = 15:1 bis 4:1.

20.  Multimetalloxidmassen nach Anspruch 1 bis 19, deren Bereiche A eine Zusammensetzung gemäß der nachfolgenden allgemeinen Formel II aufweisen

$$Mo_{12}V_{a'}X_{b'}^1 X_{c'}^2 X_{f'}^5 X_{g'}^6 O_{x'} \qquad (II),$$

mit

$X^1$ $\quad$ W und/oder Nb,
$X^2$ $\quad$ Cu und/oder Ni,

$X^5$      Ca und/oder Sr,

$X^6$      Si und/oder Al,

a'      2 bis 6,

b'      1 bis 2,

c'      1 bis 3,

f'      0 bis 0,75,

g'      0 bis 10 und

x'      eine Zahl, die durch die Wertigkeit und Häufigkeit der von Sauerstoff verschiedenen Elemente in II bestimmt wird.

21. Multimetalloxidmassen nach Anspruch 1 bis 20, die den Anteil $[B]_q$ in Form dreidimensional ausgedehnter Bereiche der chemischen Zusammensetzung B enthalten, deren Größtdurchmesser $d_B$ > 0 bis 300 μm betragen.

22. Multimetalloxidmassen nach Anspruch 1 bis 21, die den Anteil $[B]_q$ in Form dreidimensional ausgedehnter Bereiche der chemischen Zusammensetzung B enthalten, deren Größtdurchmesser $d_B$ 0,05 bis 200 μm betragen.

23. Multimetalloxidmassen nach Anspruch 1 bis 22, die den Anteil $[B]_q$ in Form dreidimensional ausgedehnter Bereiche der chemischen Zusammensetzung B enthalten, deren Größtdurchmesser $d_B$ 0,1 bis 50 μm betragen.

24. Multimetalloxidmassen nach Anspruch 1 bis 23, die den Anteil $[B]_q$ in Form dreidimensional ausgedehnter Bereiche der chemischen Zusammensetzung B enthalten, deren Größtdurchmesser $d_B$ 0,1 bis 30 μm beträgt.

25. Multimetalloxidmassen nach Anspruch 1 bis 24, deren Bereiche B Kristallite enthalten, die das Röntgenbeugungsmuster wenigstens eines der nachfolgenden Kupfermolybdate aufweisen (der Ausdruck in Klammern gibt die Quelle für den zugehörigen Röntgenbeugungsfingerabdruck wieder):

$Cu_3 (MoO_4)_2 (OH)_2$ (Lindgrenit, Karteikarte 36-405 der JCPDS-ICDD Kartei (1991)),

$Cu_4 Mo_6 O_{20}$ (A. Moini et al., Inorg. Chem. 25 (21) (1986) S. 3782 bis 3785),

$Cu_4 Mo_5 O_{17}$ (Karteikarte 39-181 der JCPDS-ICDD Kartei (1991)),

$Cu_6 Mo_5 O_{18}$ (Karteikarte 40-865 der JCPDS-ICDD Kartei (1991)),

$Cu_6 Mo_4 O_{15}$ (Karteikarte 35-17 der JCPDS-ICDD Kartei (1991)),

$Cu Mo O_4$ (Karteikarte 22-242 der JCPDS-ICDD Kartei (1991)),

$CuMoO_4$ (Russian Journal of Inorganic Chemistry 36 (7), 1991, S. 927-928, Table 1, $CuMoO_4$-III),

$Cu_{4-x} Mo_3 O_{12}$ mit x=0 bis 0,25 (Karteikarte 24-56 und 26-547 der JCPDS-ICDD Kartei (1991)),

$Cu_3 Mo_2 O_9$ (Karteikarte 24-55 und 34-637 der JCPDS-ICDD Kartei (1991)),

$Cu_2 Mo O_5$ (Karteikarte 22-607 der JCPDS-ICDD Kartei (1991)).

26. Multimetalloxidmassen nach Anspruch 1 bis 25, deren Bereiche B Kristallite B* von Oxometallaten der allgemeinen Formel III

$$Cu\ Mo_A\ W_B\ V_C\ Nb_D\ Ta_E\ O_Y \cdot (H_2O)_F \qquad (III),$$

mit

1/(A+B+C+D+E)    0,7 bis 1,3,

F               0 bis 1,

B+C+D+E      0 bis 1 und

Y               eine Zahl, die durch die Wertigkeit und Häufigkeit der von Sauerstoff verschiedenen Elemente in III bestimmt wird,

des Wolframit-Strukturtyps enthalten.

27. Multimetalloxidmassen nach Anspruch 26, mit 1/(A+B+C+D+E) = 0,85 bis 1,15 .

28. Multimetalloxidmassen nach Anspruch 26, mit 1/(A+B+C+D+E) = 0,95 bis 1,05 .

29. Multimetalloxidmassen nach Anspruch 26, mit 1/(A+B+C+D+E) = 1 .

30. Multimetalloxidmassen nach Anspruch 26 bis 29, mit F = 0.

31. Multimetalloxidmassen nach Anspruch 26 bis 30, mit B+C+D+E = 0 bis 0,5 .

32. Multimetalloxidmassen nach Anspruch 31, mit B+C+D+E = 0 bis 0,1 .

33. Multimetalloxidmassen nach Anspruch 31, mit B+C+D+E = 0 .

34. Multimetalloxidmassen nach Anspruch 1 bis 25, deren Bereiche B Kristallite B* von Oxometallaten der allgemeinen Formel IV

$$Cu\, Mo_A\, W_B\, V_c\, O_y \qquad (IV),$$

mit

| | |
|---|---|
| 1/A+B+C | 0,7 bis 1,3, |
| A,B,C | alle > 0, mit der Maßgabe, daß B+C ≤ 1 und |
| y | eine Zahl, die durch die Wertigkeit und Häufigkeit der von Sauerstoff verschiedenen Elemente in (IV) bestimmt wird, |

des Wolframit-Strukturtyps enthalten.

35. Multimetalloxidmassen nach Anspruch 1 bis 25, deren Bereiche B Kristallite B* von Oxometallaten der allgemeinen Formel V

$$Cu\, Mo_A\, W_B\, O_Y \qquad (V),$$

mit

| | |
|---|---|
| 1/A+B | 0,7 bis 1,3, |
| A,B | alle > 0, mit der Maßgabe, daß B ≤ 1 und |
| Y | eine Zahl, die durch die Wertigkeit und Häufigkeit der von Sauerstoff verschiedenen Elemente in (V) bestimmt wird, |

des Wolframit-Strukturtyps enthalten.

36. Multimetalloxidmassen nach Anspruch 1 bis 25, deren Bereiche B Kristallite B* von Oxometallaten der allgemeinen Formel VI

$$Cu\, Mo_A\, V_C\, O_Y \qquad (VI),$$

mit

| | |
|---|---|
| 1/A+C | 0,7 bis 1,3, |
| A,C | alle > 0, mit der Maßgabe, daß C ≤ 1 und |
| y | eine Zahl, die durch die Wertigkeit und Häufigkeit der von Sauerstoff verschiedenen Elemente in (VI) bestimmt wird, |

des Wolframit-Strukturtyps enthalten.

37. Multimetalloxidmassen nach Anspruch 26 bis 36, in denen der Anteil der Kristallite B*, bezogen auf die Gesamtmasse des Anteils [B]q, wenigstens 5 Gew.-% beträgt.

38. Multimetalloxidmassen nach Anspruch 26 bis 36, in denen der Anteil der Kristallite B*, bezogen auf die Gesamtmasse des Anteils [B]q, wenigstens 50 Gew.-% beträgt.

39. Multimetalloxidmassen nach Anspruch 26 bis 36, in denen der Anteil der Kristallite B*, bezogen auf die Gesamtmasse des Anteils [B]q, wenigstens 75 Gew.-% beträgt.

**40.** Multimetalloxidmassen nach Anspruch 26 bis 36, in denen der Anteil der Kristallite B*, bezogen auf die Gesamtmasse des Anteils [B]q, wenigstens 90 Gew.-% beträgt.

**41.** Multimetalloxidmassen nach Anspruch 26 bis 36, in denen der Anteil der Kristallite B*, bezogen auf die Gesamtmasse des Anteils [B]q, 95 bis 100 Gew.-% beträgt.

**42.** Multimetalloxidmassen nach Anspruch 26 und Anspruch 37 bis 38, deren Kristallite B* die Stöchiometrie $CuMoO_4$ aufweisen.

**43.** Multimetalloxidmassen der allgemeinen Formel III'

$$CuMo_A W_B V_C Nb_D Ta_E O_Y \cdot (H_2O)_F \qquad\qquad (III'),$$

mit

$1/(A+B+C+D+E)$  0,7 bis 1,3,
$F$        0 bis 1,
$B+C+D+E$    > 0 bis 1 und
$Y$        eine Zahl, die durch die Wertigkeit und Häufigkeit der von Sauerstoff verschiedenen Elemente in III bestimmt wird,

deren Strukturtyp der Wolframit-Typ ist, ausgenommen $CuWO_4$.

**44.** Multimetalloxidmassen nach Anspruch 43, mit $1/(A+B+C+D+E)$ = 0,85 bis 1,15 .

**45.** Multimetalloxidmassen nach Anspruch 43, mit $1/(A+B+C+D+E)$ = 0,95 bis 1,05 .

**46.** Multimetalloxidmassen nach Anspruch 43, mit $1/(A+B+C+D+E)$ = 1 .

**47.** Multimetalloxidmassen nach Anspruch 43 bis 46, mit F = 0.

**48.** Multimetalloxidmassen nach Anspruch 43 bis 46, mit B+C+D+E = > 0 bis 0,5 .

**49.** Multimetalloxidmassen nach Anspruch 43 bis 46, mit B+C+D+E = > 0 bis 0,1 .

**50.** Multimetalloxidmassen der allgemeinen Formel IV

$$Cu\ Mo_A\ W_B\ V_c\ O_y \qquad\qquad (IV),$$

mit

$1/A+B+C$  0,7 bis 1,3,
$A,B,C$    alle > 0, mit der Maßgabe, daß B+C ≤ 1 und
$Y$      eine Zahl, die durch die Wertigkeit und Häufigkeit der von Sauerstoff verschiedenen Elemente in (IV) bestimmt wird,

deren Strukturtyp der Wolframit-Typ ist.

**51.** Multimetalloxidmassen der allgemeinen Formel V

$$Cu\ Mo_A\ W_B\ O_Y \qquad\qquad (V),$$

mit

$1/A+B$  0,7 bis 1,3,
$A,B$    alle > 0, mit der Maßgabe, daß B ≤ 1 und
$Y$      eine Zahl, die durch die Wertigkeit und Häufigkeit der von Sauerstoff verschiedenen Elemente in (V) bestimmt wird,

deren Strukturtyp der Wolframit-Typ ist.

**52.** Multimetalloxidmassen der allgemeinen Formel VI

$$Cu\ Mo_A\ V_C\ O_Y \tag{VI},$$

mit

1/A+C     0,7 bis 1,3,

A,C     alle > 0, mit der Maßgabe, daß C ≤ 1 und

Y     eine Zahl, die durch die Wertigkeit und Häufigkeit der von Sauerstoff verschiedenen Elemente in (VI) bestimmt wird,

deren Strukturtyp der Wolframit-Typ ist.

**53.** Verfahren zur Herstellung von Multimetalloxidmassen gemäß Anspruch 43 bis 52, dadurch gekennzeichnet, daß man Quellen der die Multimetalloxidmassen konstituierenden Elemente miteinander innig mischt und das resultierende innige Gemisch in einem Überdruckgefäß im Beisein von unter überatmosphärischem Druck stehendem Wasserdampf bei Temperaturen im Bereich von > 100 bis 600°C thermisch behandelt.

**54.** Verfahren nach Anspruch 53, dadurch gekennzeichnet, daß die hydrothermale Behandlung unter solchen Bedingungen erfolgt, unter denen Wasserdampf und flüssiges Wasser zu koexistieren vermögen.

**55.** Verfahren nach Anspruch 53 oder 54, dadurch gekennzeichnet, daß die koexistierende flüssige wäßrige Phase die Gesamtmenge des Ausgangsgemisches in Suspension und/oder Lösung aufzunehmen vermag.

**56.** Verfahren nach Anspruch 53 bis 55, dadurch gekennzeichnet, daß man als Quellen ausschließlich Oxide und/oder Hydroxide einsetzt.

**57.** Verfahren nach Anspruch 53 bis 56, dadurch gekennzeichnet, daß die stöchiometrische Zusammensetzung der elementaren Konstituenten im Ausgangsgemisch derjenigen der allgemeinen Formel (III') in Anspruch 43 entspricht.

**58.** Multimetalloxidmassen, erhältlich nach einem Verfahren gemäß Anspruch 57.

**59.** Verfahren zur Herstellung einer Multimetalloxidmasse B, deren Stöchiometrie einer der allgemeinen Formeln (III'), (IV), (V) oder (VI) gemäß den Ansprüchen 43, 34, 35 und 36 genügt, dadurch gekennzeichnet, daß man von Quellen ihrer elementaren Konstituenten ein inniges Trockengemisch erzeugt und dieses bei Temperaturen von 200 bis 1000°C calciniert.

**60.** Verfahren nach Anspruch 59, dadurch gekennzeichnet, daß die Quellen der elementaren Konstituenten das Element Wolfram umfassen.

**61.** Verfahren nach Anspruch 59 oder 60, dadurch gekennzeichnet, daß das Erzeugen des innigen Trockengemisches dadurch erfolgt, daß die Quellen der elementaren Konstituenten in wäßriger Lösung und/oder Suspension miteinander vermischt werden und das wäßrige Gemisch anschließend sprühgetrocknet wird.

**62.** Verfahren nach Anspruch 59 bis 61, dadurch gekennzeichnet, daß zur Erzeugung des innigen Trockengemisches ausschließlich von in gelöster Form befindlichen Quellen der elementaren Konstituenten ausgegangen wird.

**63.** Verfahren nach Anspruch 59 bis 62, dadurch gekennzeichnet, daß zur Erzeugung des innigen Trockengemisches der elementare Konstituent Kupfer in Gestalt von in wäßriger Lösung befindlichen Kupfer-Ammoniak Komplexen eingesetzt wird.

**64.** Multimetalloxidmasse B, erhältlich nach einem Verfahren gemäß Anspruch 59 bis 63.

**65.** Verwendung von Multimetalloxidmassen gemäß Anspruch 43 bis 52 und Anspruch 58 oder 64 zur Herstellung von Multimetalloxidmassen gemäß Anspruch 1 bis 24.

66. Verfahren zur Herstellung von Multimetalloxidmassen gemäß Anspruch 1, dadurch gekennzeichnet, daß man eine Multimetalloxidmasse

$$B \quad X^7_{12} \; Cu_h \; H_i \; O_y$$

in feinteiliger Form getrennt vorbildet (Ausgangsmasse 1) und anschließend die Ausgangsmasse 1 mit geeigneten Quellen der elementaren Konstituenten einer Multimetalloxidmasse A

$$A \quad Mo_{12} \; V_a \; X^1_b \; X^2_c \; X^3_d \; X^4_e \; X^5_f \; X^6_g \; O_x$$

im gewünschten Mengenverhältnis in innigen Kontakt bringt und ein daraus resultierendes Trockengemisch bei einer Temperatur von 250 bis 450°C calciniert.

67. Multimetalloxidmassen, erhältlich nach einem Verfahren gemäß Anspruch 66.

68. Verwendung von Multimetalloxidmassen gemäß Anspruch 1 bis 42 oder gemäß Anspruch 67 als Katalysatoren zur gasphasenkatalytisch oxidativen Herstellung von Acrylsäure aus Acrolein.

69. Verfahren der gasphasenkatalytisch oxidativen Herstellung von Acrylsäure aus Acrolein, dadurch gekennzeichnet, daß als Katalysator eine Multimetalloxidmasse gemäß Anspruch 1 bis 42 oder gemäß Anspruch 67 mitverwendet wird.

**Claims**

1. A polymetal oxide material of the formula I

$$[A]_p \; [B]_q \qquad\qquad (I),$$

where

| | |
|---|---|
| A | $Mo_{12} \; V_a \; X^1_b \; X^2_c \; X^3_d \; X^4_e \; X^5_f \; X^6_g \; O_x$ (Co phase), |
| B | $X^7_{12} \; Cu_h \; H_i \; O_y$ (key phase), |
| $X^1$ | is W, Nb, Ta, Cr and/or Ce, |
| $X^2$ | is Cu, Ni, Co, Fe, Mn and/or Zn, |
| $X^3$ | is Sb and/or Bi, |
| $X^4$ | is Li, Na, K, Rb, Cs and/or H, |
| $X^5$ | is Mg, Ca, Sr and/or Ba, |
| $X^6$ | is Si, Al, Ti and/or Zr, |
| $X^7$ | is Mo, W, V, Nb and/or Ta, |
| a | is from 1 to 8, |
| b | is from 0.2 to 5, |
| c | is from 0 to 23, |
| d | is from 0 to 50, |
| e | is from 0 to 2, |
| f | is from 0 to 5, |
| g | is from 0 to 50, |
| h | is from 4 to 30, |
| i | is from 0 to 20, |
| x and y | are each a number which is determined by the valency and frequency of the elements other than oxygen in I and |
| p and q | are non-zero numbers whose ratio p/q is from 160:1 to 1:1, |

which contains the moiety $[A]_p$ in the form of three-dimensional regions A which are delimited relative to their local environment owing to their chemical composition differing from their local environment and have the chemical composition

$$A \quad Mo_{12} \; V_a \; X^1_b \; X^2_c \; X^3_d \; X^4_e \; X^5_f \; X^6_g \; O_x$$

and the moiety $[B]_q$ in the form of three-dimensional regions B which are delimited relative to their local environment owing to their chemical composition differing from their local environment and have the chemical composition

$$B \qquad X_{12}^7 \, Cu_h \, H_i \, O_y$$

the regions A and B being distributed relative to one another in the same way as in a mixture of finely divided A and finely divided B.

2. A polymetal oxide material as claimed in claim 1, where $X^1$ is W, Nb and/or Cr.

3. A polymetal oxide material as claimed in claim 1 or 2, where $X^2$ is Cu, Ni, Co and/or Fe.

4. A polymetal oxide material as claimed in any of claims 1 to 3, where $X^3$ is Sb.

5. A polymetal oxide material as claimed in any of claims 1 to 4, where $X^4$ is Na and/or K.

6. A polymetal oxide material as claimed in any of claims 1 to 5, where $X^5$ is Ca, Sr and/or Ba.

7. A polymetal oxide material as claimed in any of claims 1 to 6, where $X^6$ is Si, Al and/or Ti.

8. A polymetal oxide material as claimed in any of claims 1 to 7, where $X^7$ is Mo.

9. A polymetal oxide material as claimed in any of claims 1 to 8, where a is from 3 to 6.

10. A polymetal oxide material as claimed in any of claims 1 to 9, where b is from 0.5 to 2.5.

11. A polymetal oxide material as claimed in any of claims 1 to 10, where c is from 0 to 4.

12. A polymetal oxide material as claimed in any of claims 1 to 11, where d is from 0 to 3.

13. A polymetal oxide material as claimed in any of claims 1 to 12, where e is from 0 to 0.3.

14. A polymetal oxide material as claimed in any of claims 1 to 13, where f is from 0 to 2.

15. A polymetal oxide material as claimed in any of claims 1 to 14, where g is from 0 to 20.

16. A polymetal oxide material as claimed in any of claims 1 to 15, where h is from 6 to 24.

17. A polymetal oxide material as claimed in any of claims 1 to 15, where h is from 9 to 18.

18. A polymetal oxide material as claimed in any of claims 1 to 17, where p/q is from 20:1 to 1:1.

19. A polymetal oxide material as claimed in any of claims 1 to 17, where p/q is from 15:1 to 4:1.

20. A polymetal oxide material as claimed in any of claims 1 to 19, whose regions A have a composition of the formula II below

$$Mo_{12}V_{a'}X_{b'}^1 \, X_{c'}^2 \, X_{f'}^5 \, X_{g'}^6 \, O_{x'} \qquad\qquad (II),$$

where

$X^1$     is W and/or Nb,
$X^2$     is Cu and/or Ni,
$X^5$     is Ca and/or Sr,
$X^6$     is Si and/or Al,
a'     is from 2 to 6,
b'     is from 1 to 2,
c'     is from 1 to 3,

24

f'    is from 0 to 0.75,

g'    is from 0 to 10 and

x'    is a number which is determined by the valency and frequency of the elements other than oxygen in II.

21.  A polymetal oxide material as claimed in any of claims 1 to 20, which contains the moiety $[B]_q$ in the form of three-dimensional regions which have the chemical composition B and whose maximum diameter $d_B$ is from > 0 to 300 μm.

22.  A polymetal oxide material as claimed in any of claims 1 to 21, which contains the moiety $[B]_q$ in the form of three-dimensional regions which have the chemical composition B and whose maximum diameter $d_B$ is from 0.05 to 200 μm.

23.  A polymetal oxide material as claimed in any of claims 1 to 22, which contains the moiety $[B]_q$ in the form of three-dimensional regions which have the chemical composition B and whose maximum diameter $d_B$ is from 0.1 to 50 μm.

24.  A polymetal oxide material as claimed in any of claims 1 to 23, which contains the moiety $[B]_q$ in the form of three-dimensional regions which have the chemical composition B and whose maximum diameter $d_B$ is from 0.1 to 30 μm.

25.  A polymetal oxide material as claimed in any of claims 1 to 24, whose regions B contain crystallites which have the X-ray diffraction pattern of at least one of the following copper molybdates (the expression in parentheses gives the source of the relevant X-ray diffraction fingerprint):

$Cu_3 (MoO_4)_2 (OH)_2$ (lindgrenite, index card 36-405 of JCPDS-ICDD index (1991)),

$Cu_4 Mo_6 O_{20}$ (A. Moini et al., Inorg. Chem. $\underline{25}$ (21) (1986), 3782 to 3785),

$Cu_4 Mo_5 O_{17}$ (index card 39-181 of JCPDS-ICDD index (1991)),

$Cu_6 Mo_5 O_{18}$ (index card 40-865 of JCPDS-ICDD index (1991)),

$Cu_6 Mo_4 O_{15}$ (index card 35-17 of JCPDS-ICDD index (1991)),

$Cu Mo O_4$ (index card 22-242 of JCPDS-ICDD index (1991)),

$CuMoO_4$ (Russian Journal of Inorganic Chemistry $\underline{36}$ (7) (1991), 927-928, Table 1, $CuMoO_4$-III),

$Cu_{4-x} Mo_3 O_{12}$ where x=0 to 0.25 (index card 24-56 and 26-547 of JCPDS-ICDD index (1991)),

$Cu_3 Mo_2 O_9$ (index card 24-55 and 34-637 of JCPDS-ICDD index (1991)),

$Cu_2 Mo O_5$ (index card 22-607 of JCPDS-ICDD index (1991)).

26.  A polymetal oxide material as claimed in any of claims 1 to 25, whose regions B contain crystallites B* of oxometallates of the formula III

$$Cu\ Mo_A\ W_B\ V_C\ Nb_D\ Ta_E\ O_Y \cdot (H_2O)_F \qquad (III),$$

where

$1/(A+B+C+D+E)$ is from 0.7 to 1.3,

F              is from 0 to 1,

B+C+D+E    is from 0 to 1 and

Y              is a number which is determined by the valency and frequency of the elements other than oxygen in III,

of the wolframite structure type.

27. A polymetal oxide material as claimed in claim 26, where $1/(A+B+C+D+E)$ is from 0.85 to 1.15 .

28. A polymetal oxide material as claimed in claim 26, where $1/(A+B+C+D+E)$ is from 0.95 to 1.05 .

29. A polymetal oxide material as claimed in claim 26, where $1/(A+B+C+D+E)$ is 1 .

30. A polymetal oxide material as claimed in any of claims 26 to 29, where F is 0.

31. A polymetal oxide material as claimed in any of claims 26 to 30, where $B+C+D+E$ is from 0 to 0.5 .

32. A polymetal oxide material as claimed in claim 31, where $B+C+D+E$ is from 0 to 0.1 .

33. A polymetal oxide material as claimed in claim 31, where $B+C+D+E$ is 0 .

34. A polymetal oxide material as claimed in any of claims 1 to 25, whose regions B contain crystallites B* of oxometallates of the formula IV

$$Cu\ Mo_A\ W_B\ V_C\ O_Y \qquad\qquad (IV),$$

where

$1/A+B+C$     is from 0.7 to 1.3,

A, B and C     are all > 0, with the proviso that $B+C \leq 1$ and

Y     is a number which is determined by the valency and frequency of the elements other than oxygen in (IV),

of the wolframite structure type.

35. A polymetal oxide material as claimed in any of claims 1 to 25, whose regions B contain crystallites B* of oxometallates of the formula V

$$Cu\ Mo_A\ W_B\ O_Y \qquad\qquad (V),$$

where

$1/A+B$     is from 0.7 to 1.3,

A and B     are all > 0, with the proviso that $B \leq 1$ and

Y     is a number which is determined by the valency and frequency of the elements other than oxygen in (V),

of the wolframite structure type.

36. A polymetal oxide material as claimed in any of claims 1 to 25, whose regions B contain crystallites B* of oxometallates of the formula VI

$$Cu\ Mo_A\ V_C\ O_Y \qquad\qquad (VI),$$

where

$1/A+C$     is from 0.7 to 1.3,

A and C     are all > 0, with the proviso that $C \leq 1$ and

Y     is a number which is determined by the valency and frequency of the elements other than oxygen in (VI),

of the wolframite structure type.

37. A polymetal oxide material as claimed in any of claims 26 to 36, in which the proportion of the crystallites B* is at least 5 % by weight, based on the total mass of the moiety $[B]_q$.

38. A polymetal oxide material as claimed in any of claims 26 to 36, in which the proportion of the crystallites B* is at least 50 % by weight, based on the total mass of the moiety $[B]_q$.

39. A polymetal oxide material as claimed in any of claims 26 to 36, in which the proportion of the crystallites B* is at least 75 % by weight, based on the total mass of the moiety $[B]_q$.

40. A polymetal oxide material as claimed in any of claims 26 to 36, in which the proportion of the crystallites B* is at least 90 % by weight, based on the total mass of the moiety $[B]_q$.

41. A polymetal oxide material as claimed in any of claims 26 to 36, in which the proportion of the crystallites B* is from 95 to 100 % by weight, based on the total mass of the moiety $[B]_q$.

42. A polymetal oxide material as claimed in claim 26 and either of claim 37 or 38, whose crystallites B* have the stoichiometry $CuMoO_4$.

43. A polymetal oxide material of the formula III'

$$CuMo_A W_B V_C Nb_D Ta_E O_Y \cdot (H_2O)_F \qquad (III'),$$

where

$1/(A+B+C+D+E)$     is from 0.7 to 1.3,
F     is from 0 to 1,
B+C+D+E     is from > 0 to 1 and
Y     is a number which is determined by the valency and frequency of the elements other than oxygen in III,

whose structure type is of the wolframite type, excluding $CuWO_4$.

44. A polymetal oxide material as claimed in claim 43, where $1/(A+B+C+D+E)$ is from 0.85 to 1.15 .

45. A polymetal oxide material as claimed in claim 43, where $1/(A+B+C+D+E)$ is from 0.95 to 1.05 .

46. A polymetal oxide material as claimed in claim 43, where $1/(A+B+C+D+E)$ is 1 .

47. A polymetal oxide material as claimed in any of claims 43 to 46, where F is 0.

48. A polymetal oxide material as claimed in any of claims 43 to 46, where B+C+D+E is from > 0 to 0.5 .

49. A polymetal oxide material as claimed in any of claims 43 to 46, where B+C+D+E is from > 0 to 0.1 .

50. A polymetal oxide material of the formula IV

$$Cu\, Mo_A\, W_B\, V_C\, O_Y \qquad (IV),$$

where

$1/A+B+C$     is from 0.7 to 1.3,

A, B and C   are all > 0, with the proviso that B+C ≤ 1 and

Y   is a number which is determined by the valency and frequency of the elements other than oxygen in (IV),

whose structure type is the wolframite type.

**51.** A polymetal oxide material of the formula V

$$Cu\ Mo_A\ W_B\ O_Y \qquad (V),$$

where

1/A+B   is from 0.7 to 1.3,

A and B   are all > 0, with the proviso that B ≤ 1 and

Y   is a number which is determined by the valency and frequency of the elements other than oxygen in (V),

whose structure type is the wolframite type.

**52.** A polymetal oxide material of the formula VI

$$Cu\ Mo_A\ V_C\ O_Y \qquad (VI),$$

where

1/A+C   is from 0.7 to 1.3,

A and C   are all > 0, with the proviso that C ≤ 1, and

Y   is a number which is determined by the valency and frequency of the elements other than oxygen in (VI),

whose structure type is the wolframite type.

**53.** A process for the preparation of a polymetal oxide material as claimed in any of claims 43 to 52, wherein sources of the elements constituting the polymetal oxide material are intimately mixed with one another, and the resulting intimate mixture is thermally treated in a pressurized vessel at from > 100 to 600°C in the presence of steam under superatmospheric pressure.

**54.** A process as claimed in claim 53, wherein the hydrothermal treatment is carried out under conditions under which steam and liquid water are capable of coexisting.

**55.** A process as claimed in claim 53 or 54, wherein the coexisting liquid aqueous phase is capable of taking up the total amount of starting mixture in suspension and/or solution.

**56.** A process as claimed in any of claims 53 to 55, wherein exclusively oxides and/or hydroxides are used as sources.

**57.** A process as claimed in any of claims 53 to 56, wherein the stoichiometric composition of the elemental constituents in the starting mixture corresponds to that of the formula (III') in claim 43.

**58.** A polymetal oxide material obtainable by a process as claimed in claim 57.

**59.** A process for the preparation of polymetal oxide material B whose stoichiometry satisfies one of the formula (III'), (IV), (V) or (VI) as claimed in any of claims 43, 34, 35 and 36, wherein an intimate dry blend is produced from sources of their elemental constituents and this mixture is calcined at from 200 to 1000°C.

60. A process as claimed in claim 59, wherein the sources of the elemental constituents comprise the element tungsten.

61. A process as claimed in claim 59 or 60, wherein the intimate dry blend is produced by mixing the sources of the elemental constituents in aqueous solution and/or suspension with one another and then spray-drying the aqueous mixture.

62. A process as claimed in any of claims 59 to 61, wherein exclusively dissolved sources of the elemental constituents are used as starting materials for producing the intimate dry blend.

63. A process as claimed in any of claims 59 to 62, wherein copper in the form of copper-ammonia complexes present in aqueous solution is used for producing the intimate dry blend of the elemental constituents.

64. A polymetal oxide material B, obtainable by a process as claimed in any of claims 59 to 63.

65. Use of a polymetal oxide material as claimed in any of claims 43 to 52 and claim 58 or 64 for the preparation of polymetal oxide materials as claimed in any of claims 1 to 24.

66. A process for the preparation of a polymetal oxide material as claimed in claim 1, wherein a polymetal oxide material

$$B \quad X^7_{12} \, Cu_h \, H_i \, O_y$$

is formed beforehand separately in finely divided form (starting material 1) and starting material 1 is then brought into intimate contact with suitable sources of the elemental constituents of a polymetal oxide material A

$$A \quad Mo_{12} \, V_a \, X^1_b \, X^2_c \, X^3_d \, X^4_e \, X^5_f \, X^6_g \, O_x$$

in the desired ratio, and a resulting dry mixture is calcined at from 250 to 450°C.

67. A polymetal oxide material, obtainable by a process as claimed in claim 66.

68. Use of a polymetal oxide material as claimed in any of claims 1 to 42 or as claimed in claim 67 as a catalyst for the preparation of acrylic acid from acrolein by gas-phase catalytic oxidation.

69. A process for the gas-phase catalytic oxidative preparation of acrylic acid from acrolein, wherein a polymetal oxide material as claimed in any of claims 1 to 42 or as claimed in claim 67 is present as a catalyst.

**Revendications**

1. Masses d'oxydes multimétalliques de formule générale I

$$[A]_p \, [B]_q \qquad\qquad (I),$$

dans laquelle les variables ont les significations suivantes :

A est $Mo_{12} \, V_a \, X^1_b \, X^2_c \, X^3_d \, X^4_e \, X^5_f \, X^6_g \, O_x$ (co-phase),
B est $X^7_{12} \, Cu \, H_i \, O_y$ (phase clé),
$X^1$ est W, Nb, Ta, Cr et/ou Ce,
$X^2$ est Cu, Ni, Co, Fe, Mn et/ou Zn,
$X^3$ est Sb et/ou Bi,
$X^4$ est Li, Na, K, Rb, Cs et/ou H,
$X^5$ est Mg, Ca, Sr et/ou Ba,
$X^6$ est Si, Al, Ti et/ou Zr,
$X^7$ est Mo, W, V, Nb et/ou Ta,
a vaut de 1 à 8,
b vaut de 0,2 à 5,
c vaut de 0 à 23,

d vaut de 0 à 50,

e vaut de 0 à 2,

f vaut de 0 à 5,

g vaut de 0 à 50,

h vaut de 4 à 30,

i vaut de 0 à 20,

x et y sont des nombres qui sont définis par la valence et la fréquence des éléments de I différents de l'oxygène, et

p et q sont des nombres différents de zéro, tels que le rapport p/q soit de 160:1 à 1:1,

qui contiennent la partie $[A]_p$ sous forme de domaines A, ayant une extension tridimensionnelle, séparés de leur environnement local du fait de leur composition chimique, qui est différente de celle de leur environnement local, et ayant la composition chimique

A $\quad Mo_{12} V_a X^1_b X^2_c X^3_d X^4_e X^5_f X^6_g O_x$

et la partie $[B]_q$ sous forme de domaines B, ayant une extension tridimensionnelle, séparés de leur environnement local du fait de leur composition chimique, qui est différente de celle de leur environnement local, et ayant la composition chimique :

B $\quad X^7_{12} Cu H_i O_y$

où les domaines A, B sont répartis l'un par rapport à l'autre comme dans un mélange constitué de A finement divisé et de B finement divisé.

2. Masses d'oxydes multimétalliques selon la revendication 1, dans lesquelles $X^1$ est W, Nb et/ou Cr.

3. Masses d'oxydes multimétalliques selon la revendication 1 ou 2, dans lesquelles $X^2$ est Cu, Ni, Co et/ou Fe.

4. Masses d'oxydes multimétalliques selon les revendications 1 à 3, dans lesquelles $X^3$ est Sb.

5. Masses d'oxydes multimétalliques selon les revendications 1 à 4, dans lesquelles $X^4$ est Na et/ou K.

6. Masses d'oxydes multimétalliques selon les revendications 1 à 5, dans lesquelles $X^5$ est Ca, Sr et/ou Ba.

7. Masses d'oxydes multimétalliques selon les revendications 1 à 6, dans lesquelles $X^6$ est Si, Al et/ou Ti.

8. Masses d'oxydes multimétalliques selon les revendications 1 à 7, dans lesquelles $X^7$ est Mo.

9. Masses d'oxydes multimétalliques selon les revendications 1 à 8, dans lesquelles a vaut de 3 à 6.

10. Masses d'oxydes multimétalliques selon les revendications 1 à 9, dans lesquelles b vaut de 0,5 à 2,5.

11. Masses d'oxydes multimétalliques selon les revendications 1 à 10, dans lesquelles c vaut de 0 à 4.

12. Masses d'oxydes multimétalliques selon les revendications 1 à 11, dans lesquelles d vaut de 0 à 3.

13. Masses d'oxydes multimétalliques selon les revendications 1 à 12, dans lesquelles e vaut de 0 à 0,3.

14. Masses d'oxydes multimétalliques selon les revendications 1 à 13, dans lesquelles f vaut de 0 à 2.

15. Masses d'oxydes multimétalliques selon les revendications 1 à 14, dans lesquelles g vaut de 0 à 20.

16. Masses d'oxydes multimétalliques selon les revendications 1 à 15, dans lesquelles h vaut de 6 à 24.

17. Masses d'oxydes multimétalliques selon les revendications 1 à 15, dans lesquelles h vaut de 9 à 18.

18. Masses d'oxydes multimétalliques selon les revendications 1 à 17, dans lesquelles p/q vaut de 20:1 à 1:1.

**19.** Masses d'oxydes multimétalliques selon les revendications 1 à 17, dans lesquelles p/q vaut de 15:1 à 4:1.

**20.** Masses d'oxydes multimétalliques selon les revendications 1 à 19, dont les domaines A ont une composition selon la formule générale II ci-après :

$$Mo_{12} V_{a'} X^1_{b'} X^2_{c'} X^5_{f'} X^6_{g'} O_{x'} \qquad (II)$$

dans laquelle

$X^1$ est W et/ou Nb,
$X^2$ est Cu et/ou Ni,
$X^5$ est Ca et/ou Sr,
$X^6$ est Si et/ou Al,
a' vaut de 2 à 6,
b' vaut de 1 à 2,
c' vaut de 1 à 3,
f' vaut de 0 à 0,75,
g' vaut de 0 à 10, et
x' est un nombre qui est défini par la valence et la fréquence des éléments de II différents de l'oxygène.

**21.** Masses d'oxydes multimétalliques selon les revendications 1 à 20, qui contiennent la partie $[B]_q$ sous forme de domaines ayant une extension tridimensionnelle et ayant la composition chimique B, dont le diamètre maximale $d_B$ est compris entre > 0 et 300 $\mu$m.

**22.** Masses d'oxydes multimétalliques selon les revendications 1 à 21, qui contiennent la partie $[B]_q$ sous forme de domaines ayant une extension tridimensionnelle et ayant la composition chimique B, dont le diamètre maximale $d_B$ est compris entre 0,05 et 200 $\mu$m.

**23.** Masses d'oxydes multimétalliques selon les revendications 1 à 22, qui contiennent la partie $[B]_q$ sous forme de domaines ayant une extension tridimensionnelle et ayant la composition chimique B, dont le diamètre maximale $d_B$ est compris entre 0,1 et 50 $\mu$m.

**24.** Masses d'oxydes multimétalliques selon les revendications 1 à 23, qui contiennent la partie $[B]_q$ sous forme de domaines ayant une extension tridimensionnelle et ayant la composition chimique B, dont le diamètre maximale $d_B$ est compris entre 0,1 et 30 $\mu$m.

**25.** Masses d'oxydes multimétalliques selon les revendications 1 à 24, dont les domaines B contiennent des cristallites qui présentent l'image de diffraction aux rayons X d'au moins l'un des molybdates de cuivre ci-après (l'expression entre parenthèses donne la source de l'image de diffraction aux rayons X correspondante) :

$Cu_3(MoO_4)_2(OH)_2$ (lindgrenite, fiche 36-405 du fichier JCPDS-ICDD (1991)),
$Cu_4Mo_6O_{20}$ (A. Moini et al., Inorg. Chem. 25 (21) (1986) p. 3782 à 3785),
$Cu_4Mo_5O_{17}$ (fiche 39-181 du fichier JCPDS-ICDD (1991)),
$Cu_6Mo_5O_{18}$ (fiche 40-865 du fichier JCPDS-ICDD (1991)),
$Cu_6Mo_4O_{15}$ (fiche 35-17 du fichier JCPDS-ICDD (1991)),
$CuMoO_4$ (fiche 22-242 du fichier JCPDS-ICDD (1991)),
$CuMoO_4$ (Russian Journal of Inorganic Chemistry 36 (7), 1991, p. 927-928, Tableau 1, $CuMoO_4$-III),
$Cu_{4-x}Mo_3O_{12}$, où x vaut de 0 à 0,25 (fiches 24-56 et 26-547 du fichier JCPDS-ICDD (1991)),
$Cu_3Mo_2O_9$ (fiches 24-55 et 34-637 du fichier JCPDS-ICDD (1991)),
$Cu_2MoO_5$ (fiche 22-607 du fichier JCPDS-ICDD (1991)).

**26.** Masses d'oxydes multimétalliques selon les revendications 1 à 25, dont les zones B contiennent des cristallites B* d'oxométallates de formule générale III

$$Cu\, Mo_A\, W_B\, V_c\, Nb_D\, Ta_E\, O_y \cdot (H_2O)_F \qquad (III),$$

dans laquelle

1/(A+B+C+D+E) vaut de 0,7 à 1,3 ,

F vaut de 0 à 1,

B+C+D+E vaut de 0 à 1 , et

Y est un nombre qui est défini par la valence et la fréquence des éléments de III différents de l'oxygène,

ayant une structure du type wolframite.

27. Masses d'oxydes multimétalliques selon la revendication 26, dans lesquelles 1/(A+B+C+D+E) vaut de 0,85 à 1,15 .

28. Masses d'oxydes multimétalliques selon la revendication 26, dans lesquelles 1/(A+B+C+D+E) vaut de 0,95 à 1,05 .

29. Masses d'oxydes multimétalliques selon la revendication 26, dans lesquelles 1/(A+B+C+D+E) vaut 1 .

30. Masses d'oxydes multimétalliques selon les revendications 26 à 29, dans lesquelles F vaut 0.

31. Masses d'oxydes multimétalliques selon les revendications 26 à 30, dans lesquelles B+C+D+E vaut de 0 à 0,5 .

32. Masses d'oxydes multimétalliques selon la revendication 31, dans lesquelles B+C+D+E vaut de 0 à 0,1 .

33. Masses d'oxydes multimétalliques selon la revendication 31, dans lesquelles B+C+D+E vaut 0 .

34. Masses d'oxydes multimétalliques selon les revendications 1 à 25, dont les domaines B contiennent des cristallites B* d'oxométallates de formule générale IV

$$Cu\ Mo_A\ W_B\ V_c\ O_y \tag{IV}$$

dans laquelle

1/A+B+C vaut de 0,7 à 1,3 ,

A, B et C ont tous une valeur > 0, à la condition que B+C ≤ 1 , et

y est un nombre qui est défini par la valence et la fréquence des éléments de IV différents de l'oxygène,

ayant une structure du type wolframite.

35. Masses d'oxydes multimétalliques selon les revendications 1 à 25, dont les domaines B contiennent des cristallites B* d'oxométallates de formule générale V

$$Cu\ Mo_A\ W_B\ O_Y \tag{V}$$

dans laquelle

1/A+B vaut de 0,7 à 1,3 ,

A, B ont tous les deux une valeur > 0, à la condition que B ≤ 1 , et

Y est un nombre qui est défini par la valence et la fréquence des éléments de (V) différents de l'oxygène,

ayant une structure du type wolframite.

36. Masses d'oxydes multimétalliques selon les revendications 1 à 25, dont les domaines B contiennent des cristallites B* d'oxométallates de formule générale VI

$$Cu\ Mo_A\ V_c\ O_Y \tag{VI}$$

dans laquelle

1/A+C vaut de 0,7 à 1,3 ,

A, C sont tous les deux > 0, à la condition que C ≤ 1, et

Y est un nombre qui est défini par la valence et la fréquence des éléments de (VI) différents de l'oxygène,

ayant une structure du type wolframite.

37. Masses d'oxydes multimétalliques selon les revendications 26 à 36, dans lesquelles la proportion des cristallites B*, rapportée à la masse totale de la partie $[B]_q$, est d'au moins 5 % en poids.

38. Masses d'oxydes multimétalliques selon les revendications 26 à 36, dans lesquelles la proportion des cristallites B*, rapportée à la masse totale de la partie $[B]_q$, est d'au moins 50 % en poids.

39. Masses d'oxydes multimétalliques selon les revendications 26 à 36, dans lesquelles la proportion des cristallites B*, rapportée à la masse totale de la partie $[B]_q$, est d'au moins 75 % en poids.

40. Masses d'oxydes multimétalliques selon les revendications 26 à 36, dans lesquelles la proportion des cristallites B*, rapportée à la masse totale de la partie $[B]_q$, est d'au moins 90 % en poids.

41. Masses d'oxydes multimétalliques selon les revendications 26 à 36, dans lesquelles la proportion des cristallites B*, rapportée à la masse totale de la partie $[B]_q$, est de 95 à 100 % en poids.

42. Masses d'oxydes multimétalliques selon les revendications 26 et 37 à 38, dont les cristallites B* présentent la stoechiométrie $CuMoO_4$.

43. Masses d'oxydes multimétalliques de formule générale III'

$$Cu\ Mo_A\ W_B\ V_c\ Nb_D\ Ta_E\ O_y\ .\ (H_2O)_F \qquad (III'),$$

dans laquelle

$1/(A+B+C+D+E)$ vaut de 0,7 à 1,3 ,
F vaut de 0 à 1,
$B+C+D+E$ vaut de > 0 à 1 , et
Y est un nombre qui est défini par la valence et la fréquence des éléments de III différents de l'oxygène,

ayant une structure du type wolframite, à l'exception de $CuWO_4$.

44. Masses d'oxydes multimétalliques selon la revendication 43, dans lesquelles $1/(A+B+C+D+E)$ vaut de 0,85 à 1,15 .

45. Masses d'oxydes multimétalliques selon la revendication 43, dans lesquelles $1/(A+B+C+D+E)$ vaut de 0,95 à 1,05 .

46. Masses d'oxydes multimétalliques selon la revendication 43, dans lesquelles $1/(A+B+C+D+E)$ vaut 1 .

47. Masses d'oxydes multimétalliques selon les revendications 43 à 46, dans lesquelles F vaut 0.

48. Masses d'oxydes multimétalliques selon les revendications 43 à 46, dans lesquelles $B+C+D+E$ vaut de > 0 à 0,5 .

49. Masses d'oxydes multimétalliques selon les revendications 43 à 46, dans lesquelles $B+C+D+E$ vaut de > 0 à 0,1 .

50. Masses d'oxydes multimétalliques de formule générale IV

$$Cu\ Mo_A\ W_B\ V_c\ O_y \qquad (IV)$$

dans laquelle

$1/A+B+C$ vaut de 0,7 à 1,3 ,
A, B et C ont tous une valeur > 0, à la condition que $B+C \le 1$ , et
y est un nombre qui est défini par la valence et la fréquence des éléments de IV différents de l'oxygène,

ayant une structure du type wolframite.

**51.** Masses d'oxydes multimétalliques de formule générale V

$$Cu\ Mo_A\ W_B\ O_Y \qquad\qquad (V)$$

dans laquelle

1/A+B vaut de 0,7 à 1,3 ,
A, B ont tous les deux une valeur > 0, à la condition que B ≤ 1, et
Y est un nombre qui est défini par la valence et la fréquence des éléments de (V) différents de l'oxygène,

ayant une structure du type wolframite.

**52.** Masses d'oxydes multimétalliques de formule générale VI

$$Cu\ Mo_A\ V_c\ O_Y \qquad\qquad (VI)$$

dans laquelle

1/A+C vaut de 0,7 à 1,3 ,
A, C sont tous les deux > 0, à la condition que C ≤ 1, et
Y est un nombre qui est défini par la valence et la fréquence des éléments de (VI) différents de l'oxygène,

ayant une structure du type wolframite.

**53.** Procédé de préparation de masses d'oxydes multimétalliques selon les revendications 43 à 52, caractérisé en ce que l'on mélange intimement l'une avec l'autre des sources des éléments constituant les masses d'oxydes multimétalliques, puis on soumet à un traitement thermique à des températures comprises entre > 100 et 600°C le mélange intime obtenu, dans un récipient en surpression, en présence de vapeur d'eau sous une pression supérieure à la pression atmosphérique.

**54.** Procédé selon la revendication 53, caractérisé en ce que le traitement hydrothermal est mis en oeuvre dans des conditions dans lesquelles la vapeur d'eau et l'eau liquide peuvent coexister.

**55.** Procédé selon la revendication 53 ou 54, caractérisé en ce que la phase aqueuse liquide coexistante est à même de fixer la totalité du mélange de départ en suspension et/ou en solution.

**56.** Procédé selon les revendications 53 à 55, caractérisé en ce qu'on utilise en tant que sources exclusivement des oxydes et/ou des hydroxydes.

**57.** Procédé selon les revendications 53 à 56, caractérisé en ce que la composition stoechiométrique des constituants élémentaires du mélange de départ correspond à celle de la formule générale (III') de la revendication 43.

**58.** Masses d'oxydes multimétalliques pouvant être obtenues par un procédé selon la revendication 57.

**59.** Procédé de préparation d'une masse d'oxydes multimétalliques B, dont la stoechiométrie satisfait à l'une des formules générales (III'), (IV), (V) ou (VI) selon les revendications 43, 34, 35 et 36, caractérisé en ce qu'on produit un mélange sec intime de sources de leurs constituants élémentaires, et on calcine ce mélange à des températures de 200 à 1000°C.

**60.** Procédé selon la revendication 59, caractérisé en ce que les sources des constituants élémentaires englobent l'élément tungstène.

**61.** Procédé selon la revendication 59 ou 60, caractérisé en ce que la production du mélange sec intime est réalisée par mélange l'une avec l'autre des sources des constituants élémentaires en solution et/ou en suspension aqueuse, le mélange aqueux étant ensuite séché par atomisation.

**62.** Procédé selon les revendications 59 à 61, caractérisé en ce que, pour produire le mélange sec intime, on part exclusivement de sources sous forme dissoute des constituants élémentaires.

**63.** Procédé selon les revendications 59 à 62, caractérisé en ce que, pour produire le mélange sec intime, on utilise le constituant élémentaire cuivre sous forme de complexes cuivre-ammoniac en solution aqueuse.

**64.** Masse d'oxydes multimétalliques B pouvant être obtenue par un procédé selon les revendications 59 à 63.

**65.** Utilisation de masses d'oxydes multimétalliques selon les revendications 43 à 52 et 58 ou 64, pour préparer des masses d'oxydes multimétalliques selon les revendications 1 à 24.

**66.** Procédé de préparation de masses d'oxydes multimétalliques selon la revendication 1, caractérisé en ce qu'on forme au préalable et séparément une masse d'oxydes multimétalliques

B    $X^7_{12} Cu_h H_i O_y$

sous une forme finement divisée (masse de départ 1), puis on met en contact intime, selon le rapport pondéral souhaité, la masse de départ 1 avec les sources appropriées des constituants élémentaires d'une masse d'oxydes multimétalliques A

A    $Mo_{12} V_a X^1_b X^2_c X^3_d X^4_e X^5_f X^6_g O_x$

puis on calcine à une température de 25à à 450°C le mélange sec obtenu.

**67.** Masses d'oxydes multimétalliques pouvant être obtenues par un procédé selon la revendication 66.

**68.** Utilisation de masses d'oxydes multimétalliques selon les revendications 1 à 42 ou selon la revendication 67 en tant que catalyseurs pour préparer de l'acide acrylique à partir d'acroléine par oxydation catalysée en phase gazeuse.

**69.** Procédé de préparation par oxydation catalysée en phase gazeuse d'acide acrylique à partir d'acroléine, caractérisé en ce qu'on utilise en tant que catalyseur une masse d'oxydes multimétalliques selon les revendications 1 à 42 ou selon la revendication 67.